# EUROPÄISCHE PATENTSCHRIFT

(11) **EP 3 202 709 B1**
(45) Veröffentlichungstag und Bekanntmachung des Hinweises auf die Patenterteilung: **10.04.2019**
(21) Anmeldenummer: 16154191.7
(22) Anmeldetag: 04.02.2016
(51) Int. Cl.: B81C 99/00, B05B 1/14, B29C 33/42, B29C 45/37, A61M 15/02

(54) **ABFORMWERKZEUG MIT MAGNETHALTERUNG**
MOULDING TOOL HAVING MAGNETIC HOLDER
OUTIL DE MOULAGE AYANT UN SUPPORT MAGNÉTIQUE

(43) Veröffentlichungstag der Anmeldung: 09.08.2017
(73) Patentinhaber: Boehringer Ingelheim microParts GmbH, 44227 Dortmund (DE)
(72) Erfinder: KADEL, Klaus, 55216 INGELHEIM AM RHEIN (DE); KEYDEL, Lothar, 55216 INGELHEIM AM RHEIN (DE)
(74) Vertreter: Simon, Elke Anna Maria

(56) Entgegenhaltungen:
- EP-A1- 1 422 193
- US-A1- 2013 292 879
- ELDERS J ET AL: "DEEMO: A NEW TECHNOLOGY FOR THE FABRICATION MICROSTRUCTURES", PROCEEDINGS OF THE WORKSHOP ON MICRO ELECTRICAL MECHANICAL SYSTEMS. (MEMS). AMSTERDAM, JAN. 29 - FEB. 2, 1995, NEW YORK, IEEE, US, Bd. WORKSHOP 8, 29. Januar 1995 (1995-01-29), Seiten 238-243, XP000555275, ISBN: 978-0-7803-2504-3
- GIULIANO BISSACCO ET AL: "Precision manufacturing methods of inserts for injection molding of microfluidic systems", PRECISION MICRO/NANO SCALE POLYMER BASED COMPONENT AND DEVICE FABRICATION, 19. April 2005 (2005-04-19), XP055329617,

## Beschreibung

Die vorliegende Erfindung betrifft ein Abformwerkzeug mit Mikrostrukturen und die Verwendung des Werkzeugs bei der Herstellung mikrofluidischer Bauteile, insbesondere mikrostrukturierte Düsen, wobei die mikrofluidischen Bauteile oder mikrostrukturierten Düsen insbesondere zur Verwendung in Geräten wie vorzugsweise Zerstäubern zum Verabreichen von flüssigen, medizinischen Formulierungen geeignet sind.

US 2013/292879 A1 beschreibt ein Abformwerkzeug, das als Teil des Abformwerkzeugs eine Metallplatte mit komplementären Mikrostrukturen beinhaltet wobei die Metallplatte zumindest teilweise magnetisch im Werkzeug gehalten wird.

Ein Zerstäuber, mit dem flüssige Arzneimittelformulierungen zur Inhalation aus einem mehrere Einheiten der Formulierung enthaltenden Behälter mittels einer mikrostrukturierten Düse zerstäubt werden, ist in der WO97/12687A1 und WO09/047173A2 dargestellt. Dieser rein mechanische, miniaturisierte Hochdruckzerstäuber bildet den Ausgangspunkt für die hier dargestellte technische Entwicklung. Mit diesem Zerstäuber wird eine flüssige Arzneimittelformulierung aus einem in den Zerstäuber eingesetzten starren Behälter mit Innenbeutel, wie in der WO00/49988A2 offenbart, mittels einer durch ein Schraubgetriebe angetriebenen Kolbenpumpe aus dem Innenbeutel gefördert und mittels eines federgetriebenen Druckerzeugers in vorbestimmter Menge durch eine mikrostrukturierte Düse zu einem lungengängigen Aerosol zerstäubt. Details möglicher Mikrostrukturen für in den Zerstäubern eingesetzte Austragsdüse werden in den Schriften WO94/07607A1, WO99/16530A1, WO05/000476A1 und WO2007/101557A2 offenbart.

Ein Verfahren zur Herstellung der Düsenkörper wird in der Schrift DE4236037A1 offenbart: Die Düsenkörper setzen sich demnach aus zwei Platten zusammen, einer Bodenplatte mit grabenartigen Strukturen, wobei die Gräben einen Einlassbereich auf einer Seite der Platte mit auf der gegenüberliegenden Seite ausgeformten Düsenstrukturen verbinden. Die Bodenplatte besteht dabei aus Silizium und wird von einer unstrukturierten Platte aus Glas abgedeckt. Die Hohlräume im Düsenkörper haben dabei in der Regel rechteckige Querschnitte. Die Grabenstrukturen werden für mehrere Düsen in einem parallelen Fertigungsprozess, der ein lichtoptisches Lithographieverfahren in Verbindung mit einem ionenunterstützten reaktiven Trockenätzen beinhaltet, gleichzeitig auf einer größeren Fläche hergestellt und in einem Schritt mit der Deckelplatte durch so genanntes "anodisches Bonden" verbunden. Danach wird dieser Verbund unter dem Einsatz einer hochdrehenden Diamantkreissäge in einzelne Quader zerteilt und dabei Einlass- und Auslassöffnungen der Düsenkörper freigelegt. Bei diesem Trennprozess kann es bedingt durch die zu zerteilenden Materialien an den Seitenwänden und Kanten der Düsenkörper potentiell zu Ausbrüchen kommen, die bei der Bedienung der Säge zu vermeiden sind, da sie an der Auslassgeometrie zu ungewollten Querschnittsveränderungen führen können. Des Weiteren richtet sich die Größe des in dem geschilderten Prozess entstehenden Los (dem so genannten "Batch") u.a. nach der Größe der verwendeten Siliziumplatte und ist somit bezogen auf Massenfertigung begrenzt auf die Größe von konventionell erhältlichen Siliziumwafern.

Die vorliegende Erfindung hängt mit der Aufgabe zusammen, ein insbesondere in Bezug auf Massenfertigung gegenüber dem Stand der Technik verbessertes Verfahren zur Herstellung von mikrofluidischen Bauteilen, insbesondere Düsen für Zerstäuber anzugeben. Insbesondere soll ein Verfahren (bzw. Werkzeug zur Verwendung in einem Herstellungsverfahren) angegeben werden, mit dem im Serienbetrieb große Stückzahlen in kurzer Zeit bzw. große Bauteilmengen pro Los gefertigt werden können.

Des Weiteren soll ein insbesondere hinsichtlich seiner Tauglichkeit zur Massenfertigung verbessertes mikrofluidisches Bauteil bzw. eine dementsprechende verbesserte Düse und ein diese enthaltendes verbessertes Gerät zum Verabreichen von flüssigen medizinischen Formulierungen angegeben werden.

Die Erfindung besteht jedoch in der Verwendung eines bestimmten Abformwerkzeugs zur Herstellung eines mikrofluidischen Bauteils gemäß Anspruch 12 und in dem Abformwerkzeug selbst, das in Anspruch 1 spezifiziert wird.

Das erfindungsgemäße Abformwerkzeug kann bei der Herstellung eines mikrofluidischen Bauteils bzw. einer Düse aus Kunststoff verwendet werden, wobei die Mikrostrukturen des mikrofluidischen Bauteils bzw. der Düse zumindest teilweise in einem Abformprozess hergestellt werden. Durch eine solche Materialwahl ergibt sich ein meist kostengünstiges Ausgangsmaterial bei der Bauteilfertigung bzw. Düsenfertigung und die Möglichkeit schnelle Herstellprozesse einzurichten. Besonders bevorzugt besteht das mikrofluidische Bauteil bzw. die Düse aus einem starren Kunststoff bzw. starren Kunststoffmaterialien. Mögliche Kunststoffe für eine solche Fertigung sind beispielsweise COC, COP, Polycarbonate, Polystyrol, Polypropylen, PEEK, PMP, PA, PBT oder PMMA

Das erfindungsgemäße Abformwerkzeug weist eine Metallplatte bzw. Metallfolie mit einer komplementären Mikrostruktur auf (d.h. die komplementäre Mikrostruktur stellt ein Negativ der herzustellenden Mikrostrukturen). Die komplementäre Mikrostruktur des Abformwerkzeugs wird, wie später beschrieben, in einem galvanischen Abscheidungsprozess über einem Master-Bauteil, das besonders bevorzugt aus einem Halbleitermaterial besteht, erzeugt.

Vorzugsweise beinhaltet das Abformwerkzeug einen Stempel (insbesondere wenn der Abformprozess ein Heißprägeprozess oder Spritzprägeprozess ist) oder eine Negativform bzw. einen Formeinsatz (insbesondere wenn der Abformprozess ein Spritzgussprozess ist), wobei eine Oberfläche dieses Stempels bzw. dieser Negativform oder dieses Formeinsatzes die komplementäre Mikrostruktur aufweist. Da das Werkzeug möglichst oft zur Abformung verwendet werden soll, bevor es durch Abnutzung unbrauchbar wird, bestehen die formgebenden Oberflächen des Werkzeugs vorzugsweise aus einem robusten Metall.

Aus der DE4240857A1 ist ein Verfahren zur Herstellung eines mit Mikrostrukturen versehenen Werkzeugs zum Prägen einer Kunststoffschicht oder zum Übergießen mit Gießharz bekannt. Hierbei wird ein sogenanntes LIGA-Verfahren ("Lithographisch Galvanische Abformung") beschrieben, in dem das Werkzeug durch galvanisches Abformen einer Matrize hergestellt wird, die auf einer Seite dieselben Mikrostrukturkörper aufweist, die mit dem Werkzeug auf den Kunststoffschichten erzeugt werden sollen. Die Matrize ihrerseits wird hergestellt, indem eine metallische Basisplatte (Chrom-Nickel-Stahl) mit einer Schicht PMMA (Polymethylmethyacrylat) überzogen wird, die über Röntgenlithographie oder bei Verwendung einer anderen nicht-leitenden Schicht über andere insbesondere mechanische Verfahren derart strukturiert wird, dass in den Strukturzwischenräumen das Metall freigelegt wird. Dieser metallische Strukturgrund wird dann bei der Galvanisierung als Kathode geschaltet. Die Matrizen in solch einem Verfahren sind im Falle der Verwendung der Röntgenlithographie nicht wiederverwertbar und im Falle der mechanischen Strukturierung bei der Erzeugung kleiner Strukturen nur sehr bedingt reproduzierbar.

An dieser Stelle ergab sich als Unteraufgabe zu der oben genannten Aufgabe, dass ein Verfahren für eine verbesserte Herstellung von komplementären Mikrostrukturen auf einem Abformwerkzeug und ein entsprechendes Abformwerkzeug zum Einsatz in einem Abformprozess im nachfolgend beschriebenen Herstellungsverfahren anzugeben sind. Insbesondere soll die Herstellung einer komplementären Mikrostruktur für den Einsatz in einem Stempel bzw. einer Negativform oder Formeinsatz angegeben werden. Insbesondere sollte ein Verfahren gefunden werden, mit dem komplementäre Mikrostrukturen in Abformwerkzeugen für Spritzgussprozesse hergestellt werden, wobei auch die Herstellung der Werkzeuge selber (hinsichtlich Austausch bei Abnutzung) gut reproduzierbar ist. Auch die Lösung dieser Unteraufgabe wird im Folgenden beschrieben.

Die Herstellung der Mikrostruktur auf einem erfindungsgemäßen Abformwerkzeug kann die Verwendung eines mikrostrukturierten Master-Bauteils aus einem Halbleitermaterial, insbesondere aus hochdotierten Silizium, beinhalten. Durch die so gegebene Nutzbarkeit von aus der Halbleiter-Technologie verfügbaren Verfahren können Mikrostrukturen in Master-Bauteilen mit hoher Präzision und guter Reproduzierbarkeit erzeugt werden. Besonders bevorzugt ist hierbei die Verwendung von hochdotierten Halbleitern, insbesondere hochdotiertem Silizium, als Material für die Master-Bauteile. Es hat sich gezeigt, dass durch die Verwendung von derartigen Halbleitern mit einer guten Leitfähigkeit die entsprechenden Master-Bauteile in deutlich schlankeren Abformprozessen (hier zur Herstellung eines Abformwerkzeugs) eingesetzt werden können, als nicht-leitende Masterbauteile: Gegenüber der Verwendung von nicht-leitenden Master-Bauteilen können verschiedene Schritte im Abformprozess zur Herstellung eines Formwerkzeuges entfallen, so dass die der resultierende Prozess schneller und kostengünstiger und aufgrund seiner geringeren Komplexität potentiell auch weniger fehleranfällig sein kann. Insbesondere kann bei einer auf Galvanik basierenden Abformung des Master-Bauteils das leitfähige Master-Bauteil selbst direkt als Kathode geschaltet werden, ohne zuvor eine Metallisierung zu erhalten. Prozessschritte, die sich auf die Metallisierung beziehen, können also entfallen.

Alternativ zur Verwendung von Master-Bauteilen aus Halbleiter-Materialien können auch Master-Bauteile aus Metall verwendet werden, die mittels Mikrosystem-Technologien wie insbesondere Laserbohren oder Microfrasen strukturiert wurden. Bevorzugt werden die Mikrostrukturen im Master-Bauteil jedoch insbesondere für den geschilderten Anwendungsfall mittels Strukturierungsverfahren aus der Halbleiter-Fertigung erzeugt. Eine lithographische Strukturierung in Halbleitermaterialien bietet gegenüber den Verfahren wie Laserbohren und Microfrasen die Möglichkeit, feinere Strukturen mit gegebenenfalls höherer Reproduzierbarkeit herzustellen.

Besonders bevorzugt wird das Master-Bauteil in einem photolithographischen Verfahren in Verbindung mit einem ionenunterstützten reaktiven Trockenätzverfahren vorzugsweise aus Silizium hergestellt. Ein fakultatives Merkmal der Erfindung ist, dass das Abformwerkzeug eine Vielzahl von komplementären Mikrostrukturgruppen aufweist, wobei eine "Mikrostrukturgruppe" hier den Mikrostrukturen je eines zu erzeugenden mikrofluidischen Bauteils entspricht (d.h. das entsprechende Master-Bauteil weist die Mikrostrukturen von mehreren zu erzeugenden mikrofluidischen Bauteilen auf). Die Mikrostrukturgruppen sind bevorzugt in parallelen Reihen und Spalten auf dem Master-Bauteil bzw. auf einer von den Mikrostrukturen einmal abgesehenen flachen Oberfläche des Master-Bauteils angeordnet. Durch die Anordnung einer Vielzahl von Mikrostrukturgruppen auf dem Master-Bauteil kann man ein Abformwerkzeug (oder ein Teil eines Abformwerkzeugs) mit einer ähnlichen Vielzahl an komplementären Mikrostrukturgruppen anfertigen, so dass man bei Einsatz dieses Abformwerkzeugs in einem Abformprozess diese Vielzahl von Mikrostrukturgruppen serienmäßig in einem Fertigungslos erzeugenden kann.

Die Herstellung eines solchen Master-Bauteils mit einer Vielzahl von parallelen Mikrostrukturgruppen ist durch die Anwendung der genannten Strukturierungsverfahren aus der Halbleiter-Fertigung möglich. Diese Strukturierungsverfahren weisen eine hohe Präzision mit Strukturgenauigkeiten von einigen Mikrometern bis hinunter in den Submikrometerbereich auf. Des Weiteren sind solche für die Herstellung des Master-Bauteils eingesetzten Verfahren ihrerseits serientauglich, so dass die Master-Bauteile selbst und damit auch die Abformwerkzeuge schnell und mit einer hohen Reproduzierbarkeit neu erzeugt werden können. Somit kann beispielsweise bei einer Massenfertigung von Mikrostrukturen im Kunststoff-Spritzguss ein abgenutztes Abformwerkzeug ohne längere Maschinenstandzeiten schnell gegen ein spezifikationsgerechtes neues Werkzeug ausgetauscht werden, bzw. die Werkzeuge für so einen Austausch können leicht bevorratet werden. Ebenso können im Serienstil identische Werkzeuge für gleichzeitig ablaufende Abformprozesse produziert werden.

Ein Verfahren zur Herstellung eines Abformwerkzeugs mit komplementären Mikrostrukturen (bzw. des Teils eines Abformwerkzeugs, der komplementäre Mikrostrukturen aufweist) weist generell beispielsweise folgende, insbesondere aufeinanderfolgende Schritte auf (hier sind teilweise auch Schritte aufgeführt, die bei der bevorzugten Verwendung von Halbleiter-Materialien für das Master-Bauteil, insbesondere für die Verwendung von hochdotierten Halbleiter-Materialien entfallen können):
- Herstellen eines Master-Bauteils mit Mikrostrukturen;
- (optional) Metallisieren der Oberfläche des Master-Bauteils bzw. Aufbringen einer Metallisierungsschicht auf die Oberfläche des Master-Bauteils, welche die Mikrostrukturen aufweist, wobei zum Metallisieren vorzugsweise Gold verwendet wird, wobei besonders bevorzugt vor dem Metallisieren eine Haftschicht vorzugsweise aus Chrom aufgebracht wird;
- Galvanische Abscheidung eines Metalls (vorzugsweise Nickel oder nickelhaltig) auf der vorzugsweise metallisierten Oberfläche des Master-Bauteils (vorzugsweise wird dabei eine Schichtdicke von mindestens 500µm Metall abgeschieden);
- (bevorzugt) Randkantenbearbeitung am Werkstück, auf dem das Metall galvanisch abgeschiedenen wurde, beispielsweise durch das Erzeugen einer äußeren Kontur durch Senkerodieren in der galvanisch abgeschiedenen Metallschicht, ggf. mit anschließendem Sägen des Werkstücks im durch Senkerodieren erzeugten Spalt;
- Trennen der abgeschiedenen Metallschicht (die als Metallplatte oder Metallfolie Teil des späteren Abformwerkzeugs wird) gegebenenfalls inklusive der bei der Metallisierung erzeugten Schicht von dem Master-Bauteil, insbesondere durch nasschemisches Auflösen des Master-Bauteils;
- (optional) Gegebenenfalls Entfernen einer Haftschicht (z.B. Chrom), die bei einer vorausgegangenen Metallisierung aufgebracht wurde, insbesondere durch Ätzen (beispielsweise insbesondere selektiv mit Cer(IV)-Ammoniumnitrat)
- (optional) Randkantenbearbeitung der Metallplatte oder Metallfolie wobei zunächst ein Strukturschutz auf die komplementären Mikrostrukturen in der abgeschiedenen Metallschicht aufgebracht wird, dann die Randkanten (d.h. äußere Formgebung des hier erzeugten Teils des Abformwerkzeugs) bearbeitet werden und der Strukturschutz wieder entfernt wird;

Mit diesem Verfahren erzeugt man eine Metallplatte oder Metallfolie, welche die komplementären Mikrostrukturen aufweist und nun als Teil des Abformwerkzeugs verwendet werden kann, z.B. indem sie auf einen Stempel für Heißprägeprozesse aufgebracht wird oder Teil der inneren Wandung einer Hohlform in einem Spritzgussprozess wird.

Optional können in dem Abformwerkzeug mehrere Metallplatten oder Metallfolien mit komplementären Mikrostrukturen eingesetzt sein (dies ist z.B. möglich durch die hohe Reproduzierbarkeit in dem oben genannten Herstellungsverfahren für die Metallplatten oder Metallfolien). Auf diese Weise kann die Anzahl der Mikrostrukturgruppen, die bei einer Anwendung des Abformprozesses (z.B. Heißprägeprozess oder vor allem Spritzgussprozess) abgeformt werden (und aus denen die einzelnen mikrofluidischen Bauteile gefertigt werden), vervielfacht werden, ohne dass die Größe der Bereiche mit komplementären Mikrostrukturen im Abformwerkzeug insgesamt beispielsweise durch die Größe von Apparaturen, die in vorgeschalteten Verfahrensschritten verwendet wurden, begrenzt ist. Auf diese Weise (sowohl durch die Verwendung von Master-Bauteilen mit einer Vielzahl von Mikrostrukturgruppen als auch durch die Anordnung von mehreren Metallplatten oder Metallfolien mit komplementären Mikrostrukturen im Abformwerkzeug) kann man Abformwerkzeuge für die Erzeugung einer nahezu beliebig großen Anzahl von Mikrostrukturgruppen und damit von mikrofluidischen Bauteilen pro Fertigungslos herstellen.

Ein Merkmal des erfindungsgemäßen Abformwerkzeugs ist, dass die Metallplatte oder Metallfolie, welche die komplementären Mikrostrukturen aufweist, und ein Teil des Abformwerkzeugs ist, zumindest teilweise durch Magnete im Werkzeug gehalten wird. Besonders bevorzugt weist das Abformwerkzeug, in das beim Spritzgussprozess flüssiger Kunststoff eingespritzt wird, zwei Teile auf: einen ersten Teil, auf dem die Metallplatte oder Metallfolie mit den komplementären Mikrostrukturen magnetisch gehalten wird und einen zweiten vorzugsweise schalenartigen oder halbschalenförmigen Teil, mit dem die Metallplatte oder Metallfolie an ihren Rändern im Werkzeug festgeklemmt wird. Die komplementären Mikrostrukturen sind bei der Langzeitverwendung in Stempel oder Spritzgießprozessen und desgleichen anfällig für Abnutzung; und durch die magnetische Halterung im Werkzeug können die Metallplatte oder Metallfolie mit den komplementären Mikrostrukturen leicht ausgewechselt werden (dies gilt auch für eine zusätzliche Halterung durch Verklemmen). Hierzu ist es zweckmäßig, dass die Metallplatte oder Metallfolie aus einem ferromagnetischen Material wie z.B. Nickel oder Nickeleisen oder Nickel-Cobalt besteht.

Bevorzugt wird für die Herstellung des mikrofluidischen Bauteils bzw. eines die Mikrostrukturen aufweisenden Grundkörpers (der ein Halbzeug bei der Herstellung des mikrofluidischen Bauteils darstellt) ein Kunststoff-Spritzgussprozess verwendet. Hierfür ist ebenfalls die Verwendung von Nickel oder insbesondere Nickel-Cobalt als Material für die Metallplatte oder Metallfolie mit den komplementären Mikrostrukturen besonders geeignet, da diese Materialien auch bei höheren Temperaturen, wie sie bei Kunststoff-Spritzgussprozessen zum Einsatz kommen, noch formstabil sind.

Beim Spritzgussprozess muss die Halterung der Metallplatte oder Metallfolie mit den komplementären Mikrostrukturen den Entformungskräften widerstehen, die z.B. beim Öffnen oder Auffahren des Werkzeugs bzw. der Entnahme der abgespritzten, die Mikrostrukturen aufweisenden Grundkörper entstehen. Diese Entformungskräfte sind in der Regel so groß, dass eine Halterung basierend auf Ansaugen bzw. Unterdruck oder Vakuum nicht ausreicht. Deshalb werden in dem erfindungsgemäßen Abformwerkzeug Magnete verwendet, bevorzugt aus Samarium-Cobalt (SmCo), da es sich gezeigt hat, dass letztere auch bei höheren Temperaturen (beispielsweise im Bereich um 200°, in dem bereits einige Kunststoffe im Spritzgussprozess verarbeitet werden können) ihre magnetische Fähigkeit beibehalten, bei denen andere Magnete wie beispielsweise aus NdFeB bereits ihre magnetischen Fähigkeiten verlieren. Erfindungsgemäß werden mehrere einzelne Magnete verwendet, besonders bevorzugt mehrere einzelne SmCo-Magnete, und so nebeneinander in der Halterung der Metallplatte oder Metallfolie angeordnet, dass sich die zur Metallplatte oder Metallfolie (106f) gerichteten Pole in ihrer Polung abwechseln. Bevorzugt werden dabei flache Magnete verwendet. Bevorzugt sind die Magnete (111) so angeordnet, dass jeweils eine Seitenkante eines Magneten (111) an die Metallplatte oder Metallfolie (106f) angrenzt und dass sich die Pole von jeweils nebeneinander angeordneten Magneten (111) hinsichtlich ihrer Polung (insbesondere zueinander) abwechseln bzw. unterscheiden. Überraschend hat sich gezeigt, dass die magnetischen Haltekräfte, die auf die ferromagnetische Metallplatte oder Metallfolie wirken, durch eine solche Anordnung verstärkt werden können. Insbesondere werden hierbei rechteckige, plattenförmige SmCO-Magnete verwendet, deren Pole jeweils auf den Flachseiten liegen. Die Seitenkanten bzw. insbesondere Längskanten bilden, gegebenenfalls zusammen mit den Längskanten von optional zwischen den Magneten angeordneten Trennblechen, eine Auflagefläche für die Metallplatte oder Metallfolie.

Das mikrofluidische Bauteil bzw. die Düse (z.B. als mikrofluidisches Bauteil zur Verwendung in einem Gerät zum Verabreichen einer flüssigen medizinischen Formulierung), bei deren Herstellung das Abformwerkzeug verwendet werden kann, ist beispielsweise aus mindestens zwei Kunststoffteilen zusammengesetzt oder gebildet. Vorzugsweise sind die beiden Kunststoffteile im Wesentlichen plattenförmig und so zusammengesetzt bzw. miteinander verbunden, dass sich die mikrofluidischen Strukturen zwischen den Platten befinden. Bevorzugt sind dabei die Mikrostrukturen in Form von Gräben auf einer (vorzugsweise ansonsten ebenen) Platte ausgebildet, die von einem Deckel bedeckt wird, der (zumindest auf der der Platte zugewandten Seite) eben ist. Bei einem rechteckförmigen Kanal werden so drei Wände durch die Platte und die vierte Wand durch den Deckel (oder die Deckelplatte) gebildet. Die vorzugsweise grabenartigen Mikrostrukturen auf der Platte sind also längsseitig von dem Deckel bedeckt. Vorzugsweise sind die Platte und der Deckel aus Kunststoff fest bzw. untrennbar miteinander verbunden. Vorzugsweise besteht der Deckel zumindest teilweise aus einem starren Kunststoff.

Die Platte mit den Mikrostrukturen oder eine die Mikrostrukturen aufweisende Grundplatte wird hierbei vorzugsweise durch die in dieser Schrift geschilderten Verfahren bzw.

Abformprozess gefertigt bzw. die Mikrostrukturen werden mittels des erfindungsgemäßen Abformwerkzeugs erzeugt. Die hier geschilderten Verfahren sind auch anwendbar auf mikrofluidische Bauteile, die sich aus mehr als zwei Kunststoffteile zusammensetzen. Beispielsweise kann ein solches Bauteil aus zwei mikrostrukturierten Grundkörpern oder mikrostrukturierten Plattenkörpern bestehen, zwischen denen sich ein beidseitig ebener Plattenkörper, ggf. mit durchführenden Bohrungen befindet.

Beispielsweise erfolgt die Verbindung zwischen den mindestens zwei Kunststoffteilen bzw. die Verbindung von Platte und Deckel (bzw. Grundplatte und Deckel) durch Thermokompressionsbonden (oder Ultraschallschweißen), Laserbonden, Plasma-aktiviertes Bonden oder besonders bevorzugt durch Lösungsmittelbonden oder Polymerbonden (Bonden mit einem Zusatzmaterial wie Photolack). Die Wahl des Verfahrens richtet sich u.a. nach den Materialien von Platte und Deckel und ob beide aus dem gleichen Material bestehen. Das Verbinden der Kunststoffbauteile mittels eines Klebstoffs ist in vielen Fällen ebenfalls möglich, doch muss dabei darauf geachtet werden, das sich kein Klebstoff in den Mikrostrukturen ansammelt bzw. wieder aus diesen entfernt werden kann ohne dabei die Klebeverbindung zwischen den Platten selbst zu beeinträchtigen. Gegebenenfalls müssen vor dem Verkleben der Platten mit einem Klebstoff die Mikrostrukturen mit einem weiteren, leicht entfernbaren Material verfüllt werden.

Beim Lösungsmittelbonden wird ein Lösungsmittel (bevorzugt für mikrofluidische Bauteile mit Grundplatte und plattenförmigen Deckel aus dem gleichen Material), das geeignet ist, den verwendeten Kunststoff zumindest teilweise aufzulösen, auf mindestens einem der beiden Kunststoffteile auf der Verbindungsseite flächig verteilt. Bevorzugt wird das Lösungsmittel in einem Spincoating-Prozess (rotierender Beschichtungsprozess) auf die ebene Oberfläche des plattenförmigen Deckels bzw. auf der Deckelplatte aufgebracht. Hierbei wird mittig auf die rotierende Deckelplatte das Lösungsmittel aufdosiert. Durch dieses Spincoating-Verfahren bildet sich ein gleichmäßiger, dünner Film des Lösungsmittels auf der Deckelplatte, durch den die Oberfläche gleichmäßig angelöst wird. Anschließend wird die Grundplatte mit ihrer mikrostrukturierten Seite auf die mit Lösungsmittel bearbeitete Seite des Deckels gepresst und das resultierende Bauteil wärmebehandelt (z.B. in einem Ofen ausgeheizt). Der angelösten Kunststoff des Deckels wirkt hierbei als Kleber zwischen den beiden Kunststoffbauteilen. Durch die Behandlung des Deckels wird vermieden, die Mikrostrukturen selbst durch den Einsatz von Lösungsmittel zu verändern. Die resultierende Verbindung zwischen den beiden Kunststoffteilen ist eine flächige Verbindung. Entsprechend der individuellen Löslichkeiten der diversen Kunstoffmaterialien sind jeweils verschiedene Lösungsmittel geeignet zum Einsatz beim Lösungsmittelbonden verschiedener Kunststoffe. So ist zum Beispiel das Lösemittel Dichlormethan insbesondere geeignet zum Einsatz beim Lösungsmittelbonden von Teilen aus PMMA oder POM oder N-Methylpyrrolidon beim Bonden von Teilen aus PC oder Toluol beim Bonden von Teilen aus COP oder COC oder Xylol beim Bonden von PP.

Das Laserbonden von Kunststoffbauteilen ist in solchen Fällen bevorzugt, in denen ein Kunststoffbauteil (vorzugsweise der Deckel) transparent oder durchsichtig und das andere absorptions-empfindlich bzgl. der Wellenlänge des verwendeten Lasers ist. Die beiden Kunststoffbauteile werden beispielsweise auf einander gelegt, und der Laserstrahl wird durch das transparente Kunststoffbauteil hindurch auf die Oberfläche des darunter liegenden Bauteils gerichtet bzw. oder fokussiert. Hierbei wird beispielsweise ein Anschmelzen der Oberfläche (oder eine andere Aktivierung der Oberfläche) verursacht. Vorzugsweise durch Druckeinwirkung (Verpressen) verbinden sich die beiden Kunststoffteile an der angeschmolzenen bzw. aktivierten Oberfläche.

Alternativ können die mindestens zwei Kunststoffbauteile auch durch Plasma-aktiviertes Bonden mit einander verbunden werden. Dieses Verfahren bietet sich beispielsweise an, wenn beide Kunststoffbauteile Mikrostrukturen aufweisen, die beim Zusammensetzen des mikrofluidischen Bauteils zueinander gerichtete werden (d.h. wenn auch der Deckel mikrostrukturiert ist). Beim Plasma-aktivierten Bonden werden ein oder beide Kunststoffbauteile Plasma-behandelt, zusammengepresst und vorzugsweise wärmebehandelt (ausgeheizt). Bevorzugt erfolgt die Aktivierung mit einem atmosphärisch basierten Plasma bzw. mit einem Plasma auf Basis von Sauerstoff oder einem Sauerstoff-Edelgasgemisch (z.B. Beimischung von Argon oder Helium).

Optional wird bei Plasmaaktivierung auch eine Plasma-chemische Bearbeitung durchgeführt, bei der die Oberflächen der grabenartigen Mikrostrukturen derart verändert werden, dass sie je nach Anwendungswunsch hydrophile oder hydrophobe Eigenschaften bzw. Beschichtungen aufweisen. Dies wird dadurch erzielt, dass beispielsweise ein so genanntes Precursor-Gas in das Plasma eingeleitet wird. Das Precursor-Gas enthält beispielsweise fragmentierte Kunststoffe und regt die Polymerbildung bzw. den Aufbau von Verbindungen an oder funktionalisiert.

Bei der Herstellung des mikrofluidischen Bauteils weist z.B. die Grundplatte bzw. die mindestens eine mikrostrukturierte Platte vorzugsweise eine Vielzahl von Mikrostrukturgruppen auf, die gemeinsam von einem Deckel bedeckt und erst anschließend z.B. in konventionellen Sägeprozessen in die zu erzeugenden mikrofluidischen Bauteile (oder im Falle des Anwendungsbeispiels, Düsen) vereinzelt werden. Je nach Wahl des Materials für Grundplatte und Deckel, d.h. bei der Wahl von Kunststoffmaterialien, die z.B. aufgrund einer geringen Sprödheit beim Sägen nicht zum Splittern neigen, kann beim Sägen vorzugsweise auf zusätzliche schützende Prozessschritte wie beispielsweise ein Verfüllen der Mikrostrukturen mit einer Schutzschicht und anschließendes Auslösen / Ausspülen dieser Schutzschicht vermieden werden. Außerdem kann das Kunststoffinaterial hinreichend weich gewählt werden, so dass es sich z.B. mit Wasserstrahl-Schneidtechniken zerlegen lässt und beispielsweise auf den Einsatz von vergleichsweise teuren Diamantsägeblättern oder Energieintensiven Laserschneidprozessen verzichtet werden kann.

Zur Vereinfachung des Schneidprozesses weisen vorzugsweise (bei der Verwendung von transparenten Materialien für den Deckel) die Strukturen der abgeformten Grundplatten Schneidmarkierungen auf, anhand derer die Schnitte ausgerichtet werden, mit denen die gedeckelten Grundplatten in die einzelnen mikrofluidischen Bauteile (bzw. Düsen) zerlegt werden.

Bevorzugt ist das mikrofluidisches Bauteil eine Düse zur Zerstäubung der flüssigen medizinischen Formulierung und weist mindestens einen Düsenkanal oder einer Düsenöffnung und vorzugsweise eine Filterstruktur auf.

Vorzugsweise werden die Düsen für die Zerstäubung von medizinischen Formulierungen aus einem Kunststoff gefertigt, dessen Materialwahl sich nach der Zusammensetzung der medizinischen Formulierung richtet. Bei der Verwendung von wässrigen Formulierungen, werden hierbei vorzugsweise Materialien mit hydrophoben Eigenschaften bzw. hydrophoben Oberflächen verwendet, damit sich im Düsenauslassbereich (bzw. an Stellen, wo das mikrofluidische System mit Luft in Kontakt kommt) keine oder nur reduziert Formulierungsreste ansammeln, aus denen sich sonst (je nach Bestandteilen) z.B. bei Austrocknung Agglomerate bilden können, welche die Güte von mit den Düsen erzeugten Sprays beeinträchtigen können. In diesem Sinne wird die Verwendung eines Kunststoffs mit einer Anti-Haft-Wirkung bezogen auf die jeweiligen Substanzen der verwendeten medizinischen Formulierung bevorzugt.

Das Material der Düse wird ansonsten so gewählt, dass es beständig gegen das in der Formulierung enthaltene Lösungsmittel ist und auch chemisch inert gegenüber den anderen Bestandteilen der Formulierung ist. Der Hauptgrund dafür ist natürlich, dass bei medizinischen Anwendungen die aus der Düse austretende Formulierung in keiner Weise durch das Material der Düse verändert werden darf. Die chemische Inertheit des Düsenmaterials hat jedoch darüber hinausgehend den Effekt, dass die potentielle Bildung von Ablagerungen vermindert wird. Generell ist das Entstehen von Ablagerungen abhängig von der Zusammensetzung der Formulierung, den Löslichkeiten ihrer Bestandteile, dem Wechselwirkungspotential der Bestandteile untereinander und mit Gerätebauteilen (wie hier mit den Kanalwänden in der Düse) im Fluidkontakt und von dem Verhalten der Formulierung bei unterschiedlichen klimatischen Bedingungen insbesondere bei verschiedenen Temperaturen.

Bevorzugt werden die Düsen beispielsweise aus COC (Cyclo Olefine Copolymer), COP (Cyclo Olefine Polymer), Polystyrol, Polypropylen, PEEK, PMP (Polymethylpenten) oder PMMA hergestellt. Generell ist es möglich mikrofluidischen Bauteile beispielsweise auch aus Polycarbonaten (PC) (hohe Festigkeit, transparent und farblos) herzustellen, solche Bauteile können jedoch aus chemischer Sicht aufgrund der vielen Sauerstofffunktionen im Material und der sich daraus ergebenden Reaktivität für den Betrieb mit medizinischen Formulierungen problematisch sein. Ähnliches gilt für mikrofluidische Bauteile aus PA (Polyamiden) oder PBT (Polybutylenterephthalat) aufgrund ihrer Anfälligkeit für chemische Spaltung (PA) bzw. ihrer Empfindlichkeit gegenüber Hydrolyse (PBT).

Bei den hier dargestellten Geräten zum Verabreichen von flüssigen medizinischen Formulierungen werden bevorzugt Zerstäuber betrachtet, mit denen Flüssigkeiten über Düsenstrukturen ausgebracht, d.h. zerstäubt oder vernebelt werden.

Der Begriff "Flüssigkeit" umfasst neben reinen Flüssigkeiten und Lösungen zusätzlich Dispersionen, Suspensionen, Suslutionen (Mischungen aus Lösungen und Suspensionen) oder dergleichen. Unter dem Begriff "medizinische Formulierung" oder "Arzneimittelformulierung" sind in diesem Zusammenhang über Medikamente hinaus auch Therapeutica oder dergleichen, insbesondere also jede Art von Mitteln zur Inhalation oder sonstigen Anwendung zu verstehen.

Unter dem Begriff "Aerosol" ist insbesondere eine wolken-artige Ansammlung einer Vielzahl von Tröpfchen einer zerstäubten Flüssigkeit zu verstehen, wobei sich die Tröpfchen bevorzugt mit niedrigen Geschwindigkeiten bewegen. Insbesondere im Falle des hier betrachteten Zerstäuber hat die Tröpfchenwolke eine konische Form und eine Hauptausbreitungsrichtung, die besonders bevorzugt der Hauptströmungsrichtung der Flüssigkeit im mikrofluidischen Bauteil entspricht.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnungen. Die Zeichnungen zeigen in:
- Fig. 1: einen schematischen Schnitt eines Zerstäubers im ungespannten Zustand,
- Fig. 2: einen schematischen, um 90° gegenüber Fig.1 gedrehten Schnitt des Zerstäubers aus Fig. 1 im gespannten Zustand,
- Fig. 3: eine schematische Draufsicht auf die Mikrostruktur einer Düse zum Einbau in einen Zerstäuber.
- Fig. 4: schematische Darstellung von mehreren Teilschritten eines Verfahrens zur Herstellung eines Abformwerkzeugs mit Mikrostrukturen ausgehend von der Aufsicht auf ein schematisch dargestelltes mikrostrukturiertes Masterbauteil (Fig. 4a, mit Teilschnittansicht in Fig. 4b) über in der Regel im Querschnitt schematisch dargestellte Verfahrensschritte (Fig. 4c bis 4m) zur Herstellung des Abformwerkzeugs und einer schematischen Querschnittsansicht des Abformwerkzeugs (Fig. 4n) bis zur schematischen Querschnittsansicht eines mikrofluidischen Bauteils (bzw. einer Düse) (Fig. 4o).
- Fig. 5: eine magnetische Halteplattenanordnung für den Einsatz in einem Abformwerkzeug

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

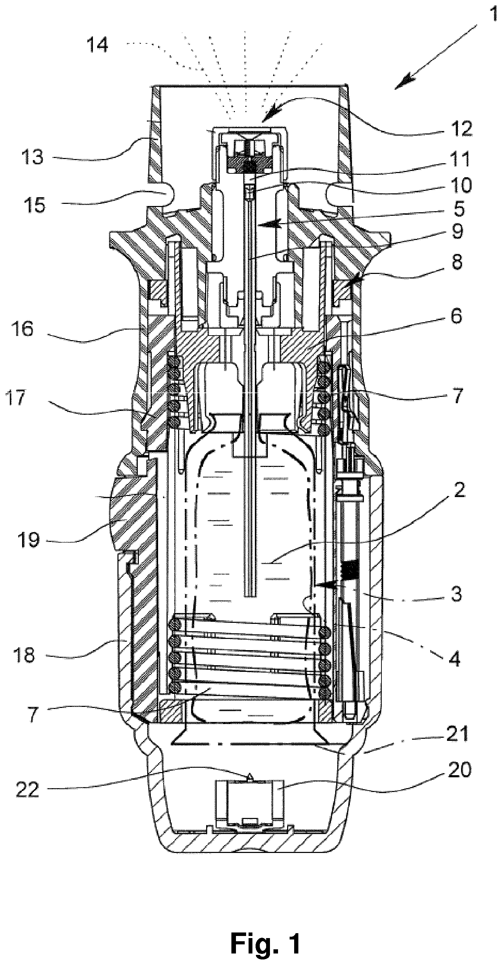

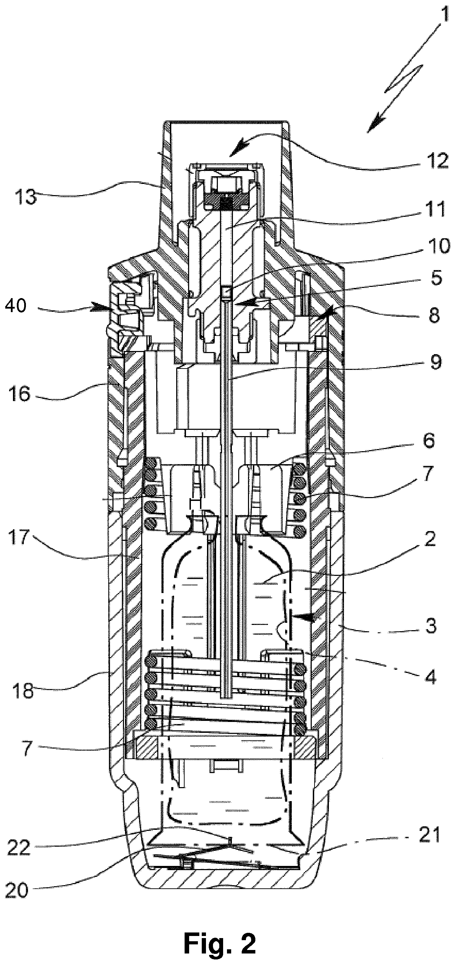

Fig. 1 und 2 zeigen in einer schematischen Darstellung ein handbetätigtes medizinisches Gerät, in dem ein mikrofluidisches Bauteil eingesetzt werden kann, das vorschlagsgemäß unter Verwendung des erfindungsgemäßen Abformwerkzeugs hergestellt wird. Bei dem Gerät aus Fig. 1 und 2 handelt es sich um einen treibgasfreien Zerstäuber (1), der pro Betätigungszyklus die jeweilig vorbestimmte Menge einer Flüssigkeit (2) bzw. einer flüssigen medizinischen Formulierung als vorzugsweise lungengängiges bzw. inhalierfähiges Aerosol (14) aus einer Düse (12) ausgibt. Dieses Aerosol (14) mit Tröpfchen mit aerodynamischen Durchmessern von vorzugsweise 0,5 bis 10 Mikrometern, insbesondere 0,5 bis 5 Mikrometer kann mit beispielsweise durch Zuluftöffnungen (15) am Mundstück (13) zuströmender Umgebungsluft von einem nicht dargestellten Benutzer eingeatmet werden, der hierzu den Zerstäuber (1) mit dem Mundstück (13) am Mund ansetzt. Beim Betrieb des Zerstäubers (1) wird unterschieden zwischen dem ungespannten Zustand mit unbefülltem Dosiervolumen in der Druckkammer (11) (Fig. 1) und dem gespannten Zustand mit befüllter Druckkammer (11) (Fig. 2).

Beim Spannen des Zerstäubers (1) wird sein Gehäuseoberteil (16) relativ zum Gehäuseinnenteil (17) und Gehäuseunterteil (18) um einen festen Drehwinkel von vorzugsweise 180° gedreht. Mittels eines innen angeordneten Schraubgetriebes wird durch die Relativdrehung eine Kolbenpumpe angetrieben, so dass eine vorbestimmte, ggf. einstellbare Menge der Flüssigkeit (2) aus dem Behälter (3) in die Druckkammer gefördert und gleichzeitig die Antriebsfeder (7) des Druckerzeugers (5) gespannt wird (Endzustand des Spannvorgangs ist in Fig. 2 dargestellt). Beim Auslösen des Zerstäubers (1), d.h. bei Betätigung eines Sperrings (8) über eine Taste (40) wird die in der Antriebsfeder (7) gespeicherte Energie des Druckerzeugers (5) freigesetzt: Der zuvor zur Flüssigkeitsförderung benutzte Hohlkolben (9) drückt nun mit geschlossenem Rückschlagventil (10) in die Druckkammer (11), so dass die durch die Hubbewegung des Hohlkolben (9) vorbestimmte Flüssigkeitsmenge von dort durch die Düse (12) ausgebracht wird. Das Gerät befindet sich nun wieder im entspannten Zustand (Fig. 1).

Der Hohlkolben (9) ist zugleich das Verbindungselement zwischen Druckkammer (11) und dem Inneren des Behälters (3). Wird der Hohlkolben (9) im Rahmen des Spannvorgangs teilweise aus der Druckkammer (11) herausgezogen, entsteht dort ein Unterdruck, durch den Flüssigkeit (2) aus dem Behälter (3) über das in dieser Situation offene Rückschlagventil (10) im Hohlkolben (9) in die Druckkammer (11) geleitet wird. Schnellt der Hohlkolben (9) beim Auslösen des Zerstäubers (1) in die Druckkammer (11) hinein, ist das Rückschlagventil (10) durch Anliegen seiner Dichtflächen am Sitz im Hohlkolben geschlossen und die Flüssigkeit in der Druckkammer (11) wird unter Druck durch einen oder mehrere Filter (mögliche Filter und Filtersysteme sind beispielsweise in der WO2012007315 beschrieben) und die Düse (12) ausgetrieben. Hohlkolben (9) und Druckkammer (11) sind gegen einander beispielsweise durch eine elastomere Dichtung abgedichtet, die insbesondere die Form eines O-Rings hat und sich in der Führungsröhre des Kolbens nahe dessen Eintritt in die Druckkammer (11) befindet; die geometrische Einbausituation dieser Dichtung, die vorzugsweise mittels eines Stützrings verpresst wird, entspricht beispielsweise der in der WO07/051536A1 beschriebenen Situation.

Im Darstellungsbeispiel ist der Hohlkolben (9) fest mit einer zum Druckerzeuger (5) gehörenden Halterung (6) für den Behälter (3) verbunden - beispielsweise angespritzt, verklebt oder verschnappt. Der Behälter (3) wird über die Halterung (6) insbesondere klemmend oder rastend so in dem Zerstäuber (1) fixiert, dass der Hohlkolben (9) in den Fluidraum des Behälters (3) eintaucht und/oder fluidisch mit der Flüssigkeit (2) im Behälter (3) verbunden wird und sie über den Hohlkolben angesaugt werden kann.

Der Behälter kann wahlweise austauschbar sein. Das Gerätegehäuse kann zu diesem Zweck so gestaltet sein, dass es aufgemacht oder teilweise abgenommen werden kann (z.B. in Form eines kappenartigen Gehäuseunterteils (18) wie in WO07/128381A1 offenbart). Der Behälter (3), der in den vorzugsweise mit einem Dosisindikator oder einem Zählwerk ausgestatteten Zerstäuber (1) eingesetzt wird, ist für die Entnahme mehrerer Dosiseinheiten ausgelegt. Dazu muss er so beschaffen sein, dass der Innendruck auch bei Flüssigkeitsentnahme im Wesentlichen unverändert bleibt, so dass beim Ansaugen immer die gleiche Menge Flüssigkeit (2) entnommen wird. Hierzu kann prinzipiell sowohl ein Behälter (3) mit starrer Behälterwand, dessen Innendruck über eine Belüftung konstant gehalten wird und wie er beispielsweise in der WO06/136426A1 beschrieben wird, als auch ein Behälter (3) mit einer flexiblen Wand verwendet werden, die sich bei Flüssigkeitsentnahme zumindest teilweise derart ins Behälterinnere verschiebt, dass durch Verkleinerung des Innenvolumens der Innendruck konstant gehalten wird. Bevorzugt werden hierbei Behälter (3), in denen die flexible Wand durch einen Beutel (4) gebildet wird, der im Wesentlichen verformbar, zusammendrückbar und/oder zusammenziehbar ausgebildet ist. Solche Behälter werden in verschiedenen Ausführungsformen in den Schriften WO00/49988A2, WO01/076849A1, WO99/43571A1, WO09/115200A1 und WO09/103510A1 beschrieben. In der dargestellten Ausführungsform weist der Zerstäuber (1) beispielsweise ein auf einer Feder (20) gelagertes Anstechelement (22) auf, das eine Belüftung im Behälterboden (21) ermöglicht.

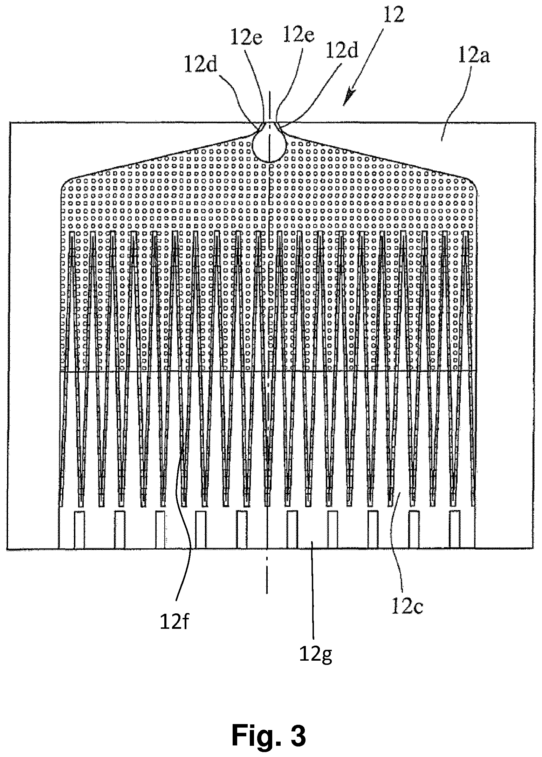

Zur Erzeugung von inhalierfähigen Aerosolen benötigen die meisten Zerstäubungskonzepte sehr klein dimensionierte Düsenstrukturen. Im Falle des Ausführungsbeispiels betragen die Abmessungen der Düsenkanäle (12d) der favorisierten mikrostrukturierten Düse (12) nur wenige Mikrometer. Bevorzugt haben die Düsenkanäle (12d) ein rechteckiges Profil mit Kantenlängen von 2 bis 10 Mikrometern (entsprechend für Kanalhöhe und Kanalbreite). Die Struktur einer für den Einsatz im Ausführungsbeispiel verwendbaren mikrostrukturierten Düse ist in Fig. 3 dargestellt. Im Beispiel beruht die Zerstäubung der Flüssigkeit mit dem Zerstäuber vorzugsweise auf der Impaktion von zwei mikroskopischen Flüssigkeitsstrahlen mit hoher Geschwindigkeit: Aus den vorzugsweise zwei Düsenkanälen (12d) bzw. den zugehörigen Düsenöffnungen (12e) treten Flüssigkeitsstrahlen derart gerichtet aus, dass sie in einem definierten Winkel aufeinandertreffen und durch die beim Aufprall wirkenden Kräfte vernebelt werden. Lagern sich bei Betrieb des Geräts in diesen Düsenkanälen (12d) Partikel an, so können die Flüssigkeitsstrahlen gegebenenfalls abgelenkt werden, so dass die Impaktion und somit die Zerstäubung nicht mehr vollständig ist bzw. im Extremfall gar nicht zustande kommt. Aus diesem Grund sollten die Partikel bereits möglichst vollständig aus der Flüssigkeit (2) herausgefiltert werden, bevor diese in die Düsenkanäle (12d) einströmt. Aus gleichem Grund wird die Düse aus einem Material hergestellt, das eine Anti-Haft-Wirkung bezogen auf die Bestanteile der zu zerstäubenden medizinischen Formulierung aufweist, damit sich insbesondere an der Flüssigkeits-Luft-Grenzfläche im Auslassbereich der Düsenkanäle (12d) keine Materialansammlungen durch Agglomeration oder Austrocknung ergeben.

Im Ausführungsbeispiel enthält das die Düse (12) bildende mikrostrukturiertes Bauteil nicht nur die eigentlichen Düsenkanäle (12d) sondern auch einen integrierten Feinfilter (12f), der auch kleinste Partikel daran hindert, in die Düsenkanäle zu gelangen. In einer favorisierten Ausführungsform setzt sich die Düse (12) bzw. das mikrostrukturierte Bauteil, aus einer mikrostrukturierten Platte (12a), und einem die Strukturen abdeckenden und fest mit der Platte (12a) verbundenen ebenen Platte (Deckel), zusammen. Bevorzugt bestehen Platte (12a) und Deckel aus Kunststoff (nicht zwingend dem gleichen), wobei die Platte (12a) bzw. eine Grundplatte, aus der die Platte (12a) herausgetrennt oder herausgeschnitten wurde, in einem Abformprozess (mit dem erfindungsmäßen Abformwerkzeug) hergestellt wurde.

Die auf diese Weise eingeschlossene Struktur bildet entlang der Strömungsrichtung mindestens eine, vorzugsweise aber mehrere Einlassöffnungen mit einem sich daran anschließenden gemeinsamen Einströmbereich (12c) und hinter einem Einströmbereich (12c) zuerst einen als Strömungsfilter ausgelegten Feinfilter (12f) und anschließend die Düsenkanäle (12d). Die Filterwirkung wird durch eine spezielle Anordnung von festen Stegen und Durchlässen erzielt. Besonders bevorzugt ist die zickzackförmige Anordnung von Stegreihen mit kleinen Durchlässen (mit deutlich kleineren Querschnitten als die Düsenöffnungen) mit fertigungsbedingt rechteckigem Profil. Die Durchlassbreiten betragen hierbei nur wenige Mikrometer - vorzugsweise werden Teilchen bis zu einer Größe von etwa 2 Mikrometern aus der Flüssigkeit entfernt, bevor diese in die Düsenkanäle eintritt und später nach Zerstäubung von einem Benutzer des Inhalators eingeatmet wird. Bevorzugt weist das mikrofluidische Bauteil stromabwärts des durch Stege und Durchlässe gebildeten Feinfilters einen gemeinsamen Ausströmbereich (vor dem Düsenauslass) auf, der optional auch weitere Strukturen oder Filterelemente beinhalten kann. Weitere Details möglicher Mikrostrukturen für die in der Düsenbaugruppe (29) eingebaute Düse (12) bzw. Feinfilter (12f) werden in den Schriften WO94/07607A1, WO99/16530A1, WO05/000476A1, WO07/101557A2 und WO08/138936A2 offenbart.

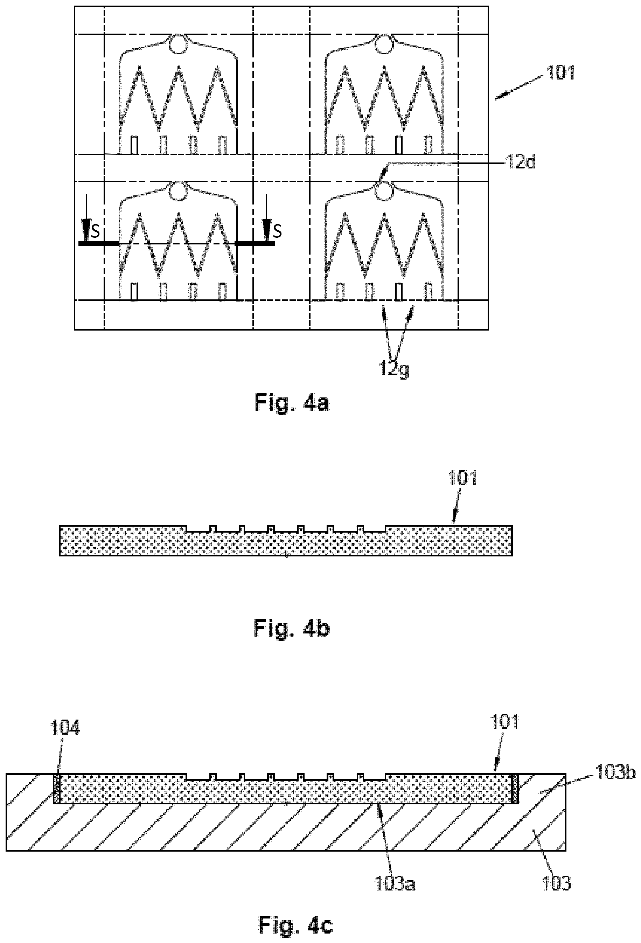

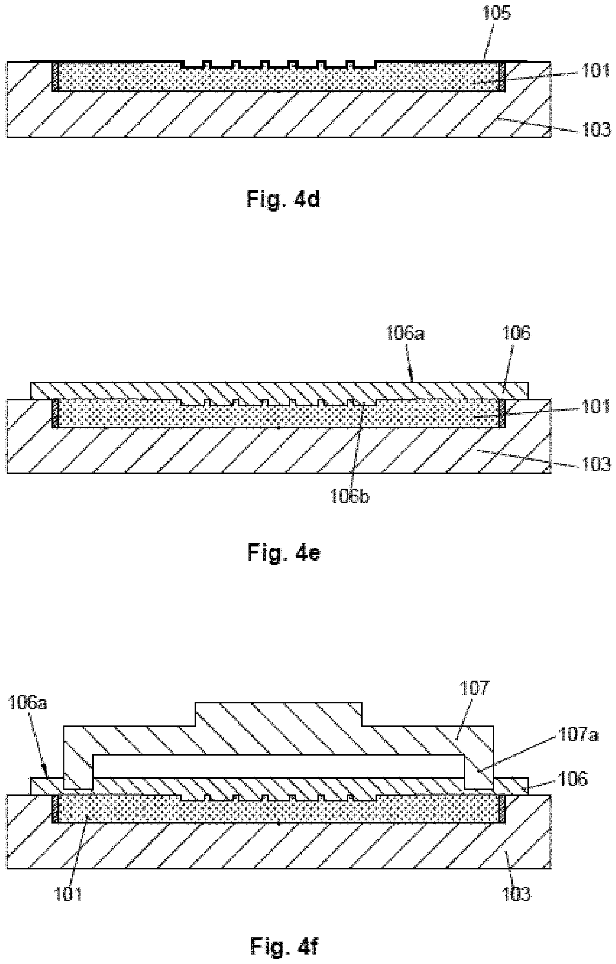

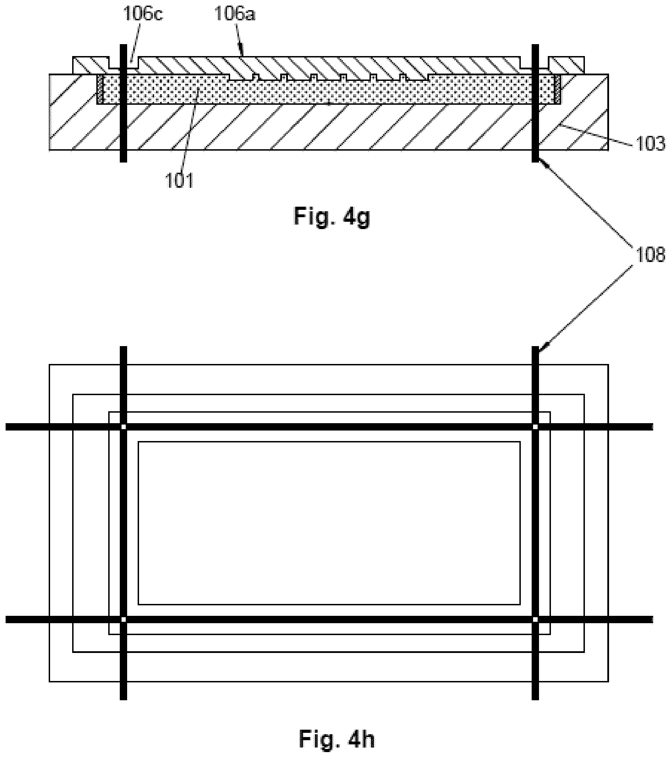

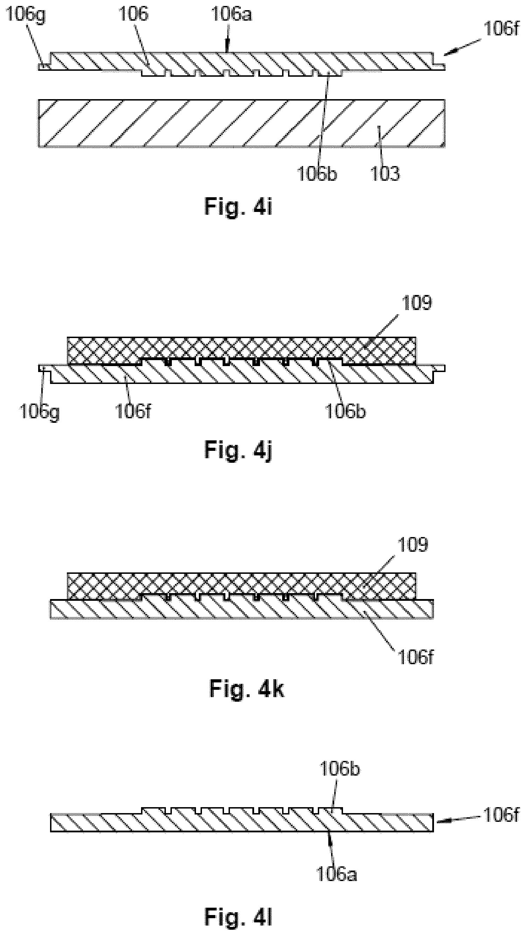

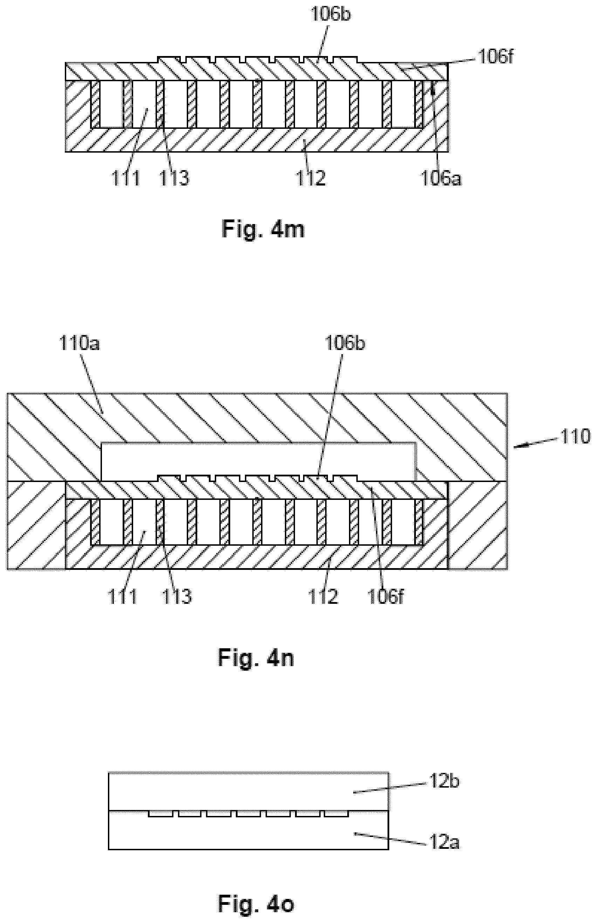

Das Gesamtsystem aus Druckerzeuger (5) mit Antriebsfeder (7), Vorfilter (27), Feinfilter (28) und Düse (12) ist vorzugsweise so aufgebaut, dass bei der Sprühnebelerzeugung nicht nur an die Lungengängigkeit angepasste Tröpfchengrößen gebildet werden, sondern dass die Sprühnebelwolke selbst so lange anhält, dass der Patient seine Einatmung leicht an sie anpassen kann. Bevorzugt werden hierbei Sprühzeiten von 0,5 bis 2 Sekunden, insbesondere von 1 bis 2 Sekunden. Die Auslegung der Mikrostrukturen, insbesondere Kanaldurchmesser und Gestaltung des Filtersystems im Zerstäuber beeinflusst hierbei die Länge der Sprühzeit. Fig. 4 zeigt schematisch ein Verfahren zur Herstellung einer Mikrostruktur für ein Abformwerkzeug und ein Verfahren, in dem anschließend mit dem Abformwerkzeug die Mikrostrukturen des mikrofluidischen Bauteils, insbesondere der Düse (12) bzw. die mikrostrukturierte Platte (12a), erzeugt werden. Gegenüber dem in Fig. 3 gezeigten Ausführungsbeispiel des mikrofluidischen Bauteils, sind die in Fig. 4 dargestellten Mikrostrukturen zur besseren Erläuterung des Herstellverfahrens rein schematisch bzw. deutlich vereinfacht dargestellt.

Bei dem hier gezeigten Verfahren wird ein so genanntes Master-Bauteil (101) verwendet, das im Wesentlichen die gleichen Mikrostrukturen wie das im Serienprozess zu erzeugende mikrofluidische Bauteil aufweist. Die exakte Dimensionierung der Strukturen insbesondere von Kanaltiefen und Kanalbreiten weicht unter Umständen etwas von den final zu erzeugenden Strukturen ab, da spätere Abformungsschritte und, je nach verwendeten Materialien, damit verbundene Materialschrumpfungen berücksichtigt werden müssen. Die Strukturhöhen im Master-Bauteil (101) liegen zwischen 2 und 40 µm, im Allgemeinen zwischen etwa 3 und 20 µm, vorzugsweise zwischen etwa 4 und 14 µm, insbesondere zwischen etwa 5 und 8 µm.

Das Master-Bauteil (101) weist vorzugsweise, wie schematisch in Fig. 4a (und in Fig. 4b in einer Schnittansicht entsprechend des Teilschnitts S-S aus Fig. 4a) dargestellt, nicht nur die Mikrostrukturen eines zu erzeugenden mikrofluidischen Bauteils sondern mehrerer vorzugsweise identischer mikrofluidischer Bauteile nebeneinander auf (es ist auch die Fertigung verschiedener Mikrostrukturen nebeneinander möglich, die würde jedoch weniger zu serientauglichen Massenproduktionsprozessen passen, da dann später ein eine Trennung verschiedenartiger Bauteile in den Prozessen berücksichtigt werden muss). Die den einzelnen zu erzeugenden mikrofluidischen Bauteilen entsprechenden Mikrostrukturen (bzw. Mikrostrukturgruppen (102)) sind auf dem Master-Bauteil (101) alle in gleicher Ausrichtung angeordnet. Im Falle des in Fig. 4 a schematisch dargestellten Master-Bauteils (101) zeigen so beispielsweise alle den Einlassöffnungen (12g) entsprechenden Strukturen in eine Richtung und alle den Düsenauslässen entsprechenden Strukturen (gebildet durch Düsenkanäle (12d)) befinden sich dem entgegengesetzt. Die Mikrostrukturgruppen (102) (d.h. die den einzelnen zu erzeugenden mikrofluidischen Bauteilen entsprechenden Mikrostrukturen) sind in mehreren parallelen Reihen und Spalten zu einander angeordnet, so dass eine spätere Vereinzelung z.B. mit Hilfe von Sägeschnitten geradlinig zwischen den Mikrostrukturgruppen (102) erfolgen kann.

Vorzugsweise wird das Master-Bauteil (101) aus einem Halbleitermaterial, wie beispielsweise Gallium-Arsenid oder Silizium, bevorzugt aber aus einem hochdotierten Silizium (insbesondere in einkristalliner Form) hergestellt (hierdurch können einige der Prozessschritte aus dem in Fig. 4c bis Fig4l dargestellten Schema entfallen, das allgemein für die Verwendung von Master-Bauteilen aus beliebigen Materialien aufgestellt wurde). Besonders bevorzugt wird ein Siliziumwafer verwendet, da ein solcher eine geringe Oberflächenrauigkeit und eine ausreichende Ebenheit und Parallelität aufweist. Bevorzugt wird das vorzugsweise plattenförmige Material bzw. der Siliziumwafer in einem an sich aus der Halbleitertechnologie bekannten photolithographischen Verfahren in Verbindung mit einem ionenunterstützten reaktiven Trockenätzverfahren (ein so genanntes RIE-Verfahren) strukturiert. Vorzugsweise wird auf der Oberfläche des Wafers eine dünne Schicht aus Silizium thermisch oxidiert. Die Oxidschicht dient später als Maskierung zum Ätzen der Kanalstrukturen. Auf diese Oxidschicht wird im Schleuderverfahren eine lichtsensitive Kunststoffschicht aufgetragen und verfestigt. Unter Verwendung einer Maske (vorzugsweise aus Chrom-Glas) wird in einem Stepper-Prozess die Kunststoffschicht schrittweise belichtet, wobei die in der Maske zweidimensional enthaltenen Strukturen vorzugsweise verkleinert auf der Kunststoffschicht abgebildet werden (Beispielsweise in Aspektverhältnissen von 5:1 oder 10:1). (Alternativ ist auch eine lichtoptisch Übertragung der Struktur mittels Kontaktkopie über eine Maske im Maßstab 1:1 in diese Kunststoffschicht möglich, aber verkleinernde Abbildungsverhältnisse ermöglichen die Erzeugung kleinerer Strukturen und eine nichtberührende Maskenführung erhöht die Lebensdauer der Maske). Die derart belichtete Kunststoffschicht wird anschließend entwickelt, so dass entsprechend der Belichtung Strukturen im Kunststoff entstehen. Die Kunststoffstrukturen dienen im nächsten Prozessschritt als Maskierung für die Strukturierung der Siliziumdioxidschicht. Diese Strukturierung erfolgt vorzugsweise durch reaktives Ätzen mit Ionen, aber alternativ ist auch die Anwendung von naßchemischen Ätzverfahren möglich. Am Ende des Strukturierungsvorgangs ist der Kunststoff vollständig abgetragen und es befinden sich auf dem Siliziumwafer U-förmige oder rechtwinklige, kastenförmige Kanalstrukturen, die jedoch in Draufsicht nahezu beliebige Flächengeometrien aufweisen können.

Optional kann statt diesem einstufigen Strukturierungsprozesses auch ein mehrstufiger Prozess verwendet werden, um verschiedene Strukturtiefen im Master-Bauteil (101) zu erzeugen. So kann zum Beispiel gewünscht sein, dass die Durchlässe der Feinfilter (12f) eine geringere Tiefe als der vorangegangene Einströmbereich (12c) und/oder der sich anschließende Ausströmbereich haben. Zum einen wird dadurch die Filterwirkung des Feinfilters (12f) verstärkt, da nun auch asymmetrische Partikel mit Längenausdehnungen größer der Durchlassbreite besser aufgehalten werden. Auch die Erzielung einer antibakteriellen Filter-Wirkung ist hier in Analogie zu den in WO08/138936A2 beschriebenen Strukturen möglich. Zum anderen wird die Entformbarkeit in späteren Prozessschritten (insbesondere bei der späteren Entformung von im Spritzguss erzeugten mikrofluidischen Bauteilen aus mit dem Master-Bauteil (101) erzeugten Abformwerkzeugen) hinsichtlich der vergleichsweise engen Strukturen verbessert.

Fig. 4c bis 4l zeigt schematisch ein Verfahren, wie anhand eines Master-Bauteils (101) aus Fig. 4a bzw. 4b eine Mikrostruktur für ein Abformwerkzeug erzeugt wird. In dem hier dargestellten allgemein gültigeren Schema kann das Masterbauteil aus einem beliebigen Material, wie beispielsweise einem Kunstoffmaterial (z.B. Polycarbonat), gefertigt sein; das Schema enthält auch Schritte, die durch eine unzureichende Leitfähigkeit des Master-Bauteils notwendig werden. Bei dem hier vorgestellten Verfahren wird die Mikrostruktur des Master-Bauteils (101) galvanisch mit einem Metall abgeformt, wobei insbesondere die ganze die Mikrostruktur aufweisende Oberfläche des Master-Bauteils mit galvanisch abgeschiedenen Metall überdeckt wird. Um anschließend das Abformwerkzeug zu erhalten muss das Master-Bauteil (101) vollständig entfernt werden; das spätere Abformwerkzeug besteht im Bereich der Mikrostrukturen vorzugsweise ausschließlich aus dem aufgalvanisierten Metall.

Fig. 4c zeigt wie das Master-Bauteil (101) auf eine Stützplatte (103) aufgesetzt bzw. eingelegt oder eingesetzt ist. Diese Stützplatte (103) weist vorzugsweise eine Vertiefung (103a) passend zum Master-Bauteil (101) auf, so dass die Höhe des Rands (103b) der Stützplatte (103) im Randbereich des in die Vertiefung (103a) eingesetzten Master-Bauteil (101) vorzugsweise gleichauf ist mit der Oberfläche des eingesetzten Master-Bauteils (101) (d.h. die Tiefe der Vertiefung (103a) entspricht vorzugsweise der Dicke (101a) des Master-Bauteils (101)). Das Material und die Dicke der Stützplatte (103) (unterhalb der Vertiefung (103a)) müssen so gewählt sein, dass sie eine für den späteren galvanischen Prozess geeignete mechanische Stabilität aufweisen. Bei diesem Prozess entstehen thermische Spannungen, die insbesondere bei zu dünnen Stützplatten (103) (z.B. im Falle von Kupfer dünner als 8 mm) zu Verformungen führen können, durch die das so hergestellte Abformwerkzeug nicht mehr eben ist und damit wiederum erzeugte mikrofluidische Strukturen entsprechend schlecht gedeckelt werden können. Bevorzugt werden Metallplatten einer Dicke von mindestens 8 mm eingesetzt, da sich Metall sehr genau plan bearbeiten lässt. Für eine galvanische Abscheidung von Nickel oder Nickel-Eisen wird vorzugsweise Kupfer als Material für die Stützplatte (103) verwendet. Vorzugsweise wird beim Einlegen des Master-Bauteils (101) in die Stützplatte (103) ein Klebstoff verwendet, so dass das Master-Bauteil (101) in der Vertiefung (103a) der Stützplatte (103) fixiert wird. Dabei oder zusätzlich wird der seitliche (spaltförmige) Zwischenraum zwischen Master-Bauteils (101) und Rand (103b) der Stützplatte (103) mit einer Füllmasse (104) aufgefüllt wird, beispielsweise mit Epoxydharz. Dies dient dazu, dass bei der anstehenden Metallisierung (Ergebnis rein schematisch dargestellt in Fig. 4d) die aufgebrachte, vorzugsweise leitfähige Metallisierungsschicht (105) nicht an der Stelle des Zwischenraums abreißt bzw. dort keine Risse aufzeigt, sondern eine durchgängige Schicht bildet (in Gegensatz zur schematischen Darstellung in Fig. 4d ist die Metallisierungsschicht (105) aber um ein Vielfaches dünner als das Master-Bauteil (101) und bedeckt Höhen, Tiefen und Wände der Mikrostrukturen ohne eine einzige Ebene zu bilden).

Die Notwendigkeit, eine Stützplatte (103) und damit die anhand von Fig. 4c erläuterten Prozessschritte zu verwenden, entfällt bei der Verwendung von strukturierten Halbleiterplatten, wie Siliziumwafern, da sie durch ihre äußere Form und durch ihre ebenen Oberflächen und vor allem ihre Stabilität direkt weiter prozessierbar sind (Bei der Darstellung eines rein auf die Verwendung von Halbleiter-Masterbauteilen bezogenen Verfahrens würde die Darstellung der Stützplatte (103) in Fig. 4d bis 4i (soweit anwendbar) entfallen). Vorzugsweise besteht die Metallisierungsschicht (105) aus Chrom und Gold, wobei beides beispielsweise in einem Sputterprozess oder einem Verdampfungsverfahren aufgebracht wird. Das Chrom dient hierbei als Haftschicht zwischen dem Master-Bauteil (101) (vorzugsweise aus Silizium) und dem Gold. Die Schichtdicken betragen dabei beispielsweise 20-30 nm für Chrom und 50-200 nm für Gold. Die Metallisierungsschicht (105) bzw. die Goldschicht dient bei dem anschließenden Galvanik-Prozessschritt als Elektrode.

In dem bevorzugten Fall, dass ein hochdotiertes Halbleitermaterial, insbesondere hochdotiertes Silizium für das Master-Bauteil (101) verwendet wird (der Halbleiter also eine hohe Leitfähigkeit aufweist), können die (beispielsweise in Fig. 4d dargestellte) Metallisierungen und beispielsweise die durch das Aufsputtern von Gold entstehenden Kosten entfallen. Das Master-Bauteil selbst würde dann direkt (insbesondere ohne irgendeine weitere Behandlung nach seiner Herstellung) in einem konventionellen Galvanik-Prozess als Abscheidungselektrode geschaltet werden. Je nach Verwendung des in dem gesamten Prozess zu erzeugenden Teil eines Abformwerkzeugs kann die Metallisierung mit einem inerten Metall wie Gold jedoch auch zusätzliche Vorteile haben, da diese Schicht eine Oberfläche im späteren Abformwerkzeugs bildet und je nach abzuformendem Material die Abformung an dieser strukturierten Oberfläche durch die inerten Beschichtung verbessert sein kann.

Fig. 4e zeigt das Master-Bauteil (101) nach der galvanischen Abscheidung einer überdeckenden Metallschicht (106) (vorzugsweise aus Nickel oder Nickel-Eisen oder Nickel-Cobalt) (Die gegebenenfalls auf dem Master-Bauteil (101) aufgebrachte Metallisierungsschicht (105) ist aufgrund ihrer Dünne in dieser Abbildung nicht dargestellt, würde sich bei Bedarf aber zwischen Master-Bauteil (101) und Metallschicht (106) befinden). Bei dieser Galvanisierung wird die metallisierte Stützplatte (103) mit eingelegtem Master-Bauteil (101) in ein Galvanik-Bad, insbesondere gebildet durch eine nickelhaltige Elektrolytlösung (beispielsweise eine Nickelsulfamat-Elektrolytlösung) in einem geeigneten Gefäß, eingesetzt und die voraus aufgebrachte Metallisierungsschicht (105) wird als Elektrode angeschlossen bzw. als Kathode geschaltet. Im Falle der vorzugsweise Verwendung eines Master-Bauteils aus einem hochdotierten Halbleitermaterial wird dieses vorzugsweise direkt in das Galvanik-Bad bzw. in eine passgenaue Verschalung, in welcher unter Verwendung einer vorzugsweise nickelhaltigen Elektrolytlösung der Galvanik-Prozess durchgeführt wird, eingesetzt und elektrisch angeschlossen, damit es beim Prozess eine Kathode bildet.

Aus Gründen der mechanischen Stabilität ist die galvanisch abgeschiedene Schicht, die später Teil des Abformwerkzeugs sein soll, vorzugsweise mindestens 500µm, besonders bevorzugt mindestens 1mm dick (zur Verwendung als Folie, bei der Herstellung eines selbsttragenden nickelhaltigen Werkzeugs ist eine Schichtdicke im Bereich 5 mm bis 8 mm bevorzugt). Prinzipiell können mittels der galvanischen Abformung Werkzeuge mit Mikrostrukturhöhen von 2-1000 µm erzeugt werden, die freien Zwischenräume, die zwischen den Mikrostrukturen vorhanden sind, können ca. 2µm bis einige mm betragen. Insbesondere können mit dieser Technik Strukturen bis zu Aspektverhältnissen von 1:50 (bezogen auf Breite zu Tiefe der Struktur im µm-Bereich). Im betrachteten Anwendungsbereich (Herstellung von Strukturen für mikrofluidische Bauteile, insbesondere Düsen) wird (je nach Strukturdetail) bevorzugt allerdings nur mit Aspektverhältnissen im Bereich von 1:4 bis 250:1 gearbeitet.

Für den hier bevorzugten Prozess (galvanische Abscheidung von einer vergleichsweise dünnen nickelhaltigen Folie auf einem an sich vergleichsweise ebenen Master-Bauteil mit Strukturtiefen, die sich lediglich im einstelligen Mikrometerbereich befinden) zeigt die auf dem Master-Bauteil bzw. auf der Metallisierungsschicht galvanisch abgeschiedene Schicht eine für eine weitere Prozessierung bzw. Weiterbearbeitung hinreichende Ebenheit der Oberfläche auf.

Dies ist unter Umständen jedoch insbesondere bei der Erzeugung dickerer (selbsttragender) nickelhaltigen Schichten z.B. mit gröberen Strukturen nicht unbedingt der Fall, so dass in einem solchen, hier nicht favorisierten Fall gegebenenfalls ein weiterer Prozessschritt aufgenommen werden sollte, in dem einer ebene Oberfläche (106a) auf dem galvanisch abgeschiedenen Metall, insbesondere durch Abschleifen der Oberfläche des galvanisch abgeschiedenen Metalls, erzeugt wird. bei dieser Glättung der Oberfläche der Metallschicht (106) auf der dem Master-Bauteil (101) abgewandten Seite sollte allerdings zur Wahrung der im späteren Verlauf benötigten Stabilität eine bestimmte Mindestschichtdicke des Metalls auf der gesamten Oberfläche verbleiben (Ergebnis zu sehen in Fig. 4e). (Beim Einebnen bzw. Abschleifen wird die der erzeugten komplementären Mikrostruktur (106b) entgegengesetzte Oberfläche bearbeitet; die bei der galvanischen Abscheidung im Metall erzeugten komplementären Mikrostrukturen (106b) werden durch das Abschleifen nicht verändert).

Fig. 4f zeigt einen bevorzugten Prozessschritt, wie vorzugsweise spaltförmige Markierungen insbesondere in Randnähe der ebenen Oberfläche (106a) aufgebracht werden, wobei die Markierungen der weiteren Bearbeitung des Werkstücks dienen (das "Werkstück" wird im Darstellungsbeispiel zu diesem Zeitpunkt vorwiegend aus Stützplatte (103), Master-Bauteil (101) und Metallschicht (106), im besonders bevorzugten Beispiel beispielsweise nur durch Master-Bauteil (101) und Metallschicht (106) gebildet). Bevorzugt sind diese Markierungen Spalte (106c), die später der Führung von Sägeblättern in einem Sägeprozess dienen. Bevorzugt werden die Spalte (106c) wie in Fig. 4f schematisch angedeutet in einem Senkerodierprozess erzeugt, bei dem eine Elektrode (107) mit einer vorstehenden Kontur (107a) in einem Dielektrikum an die ebene Oberfläche (106a) der Metallschicht (106) herangeführt wird. Im Senkerodierprozess wird durch Anlegen einer elektrische Spannung zwischen Elektrode (107) und Metallschicht (106) (oder zwischen Elektrode (107) und Master-Bauteil (101) bei der Verwendung eines Master-Bauteils aus einem Metall oder einem hochdotierten Halbleitermaterial) einer elektrischen Entladung erzeugt, durch die an den durch die vorstehende Kontur (107a) vorgegebenen Stellen Material von der ebenen Oberfläche (106a) abgetragen wird. Im Darstellungsbeispiel in Fig. 4f, 4g und 4h wird eine Kontur (107) in Form einer Rechteckumrandung verwendet, so dass auf der ebenen Oberfläche (106a) Spalte (106c) erzeugt werden, die ein umlaufendes Rechteck bilden. In diesen Spalten (106c) werden in einem anschließenden Sägeprozess Sägeblätter geführt. Die Sägelinien (108) für diesen Sägeprozess sind dabei beispielsweise in Fig. 4g und 4h skizziert. Die Spalte (106c) geben im Darstellungsbeispiel die Lage der anschließend am Werkstück erzeugten Schnittkanten vor. Vorzugsweise wird nur ein rechteckförmiger Zuschnitt des Werkstücks vorgenommen und das später erzeugte Teil des Abformwerkzeugs weist die komplementären Mikrostrukturen (106b) zu mehreren Mikrostrukturgruppen (102) auf. Es ist jedoch möglich (wenn auch im Hinblick auf eine spätere Massenfertigung nicht zweckdienlich) Konturen (107) beispielsweise in Form mehrerer nebeneinanderliegender Rechtecke zu verwenden und so beim Sägen bereits eine Vereinzelung der Mikrostrukturgruppen (102) in ihrer komplementären Form vorzunehmen. Der sich anschließende konventionelle Sägeprozess (z.B. Wasserstrahlschneiden, Rotationssägen etc.) wird entsprechend der zu durchtrennenden Materialien (z.B. von Stützplatte und Master-Bauteil) gewählt.

Bei der Verwendung von konventionell erhältlichen Siliziumwafern für die Masterbauteile, können an dieser Stelle die der Randbearbeitung dienenden Prozessschritte (schematisch dargestellt in Fig. 4f bis 4g und Fig. 4j bis 4k) optional entfallen, wenn die abgeschiedene Metallschicht (106) bzw. eine durch sie gebildete Folie (schematisch dargestellt in Fig. 4l) sich durch ihre äußere Form bereits für den späteren Einsatz in Abformwerkzeugen eignet. Beispielsweise kann man bei der Verwendung von typischerweise runden, scheibenförmigen Siliziumwafer für die Master-Bauteile bei entsprechend passender geometrischen Auslegung des Galvanik-Bades kreisförmige Folien gleichen Durchmessers erzeugen, die in Abformwerkzeuge eingespannt oder randseitig eingeklemmt werden können. Das Produkt, das mit dem resultierenden Abformwerkzeug hergestellt wird, kann dann durchaus von der Kreisform abweichende Randkonturen aufweisen, da z.B. im Falle eines Spritzgusswerkzeug nur ein Teil der Folie eine Seite der auszuspritzenden Form darstellen kann (und der nicht benötigte Teil der Folie nicht ans Innere der Werkzeugform angrenzt, sondern z.B. einen Klemmbereich bildet).

Durch die Randbearbeitung, insbesondere durch das Sägen, wird (bezogen auf Verfahren, in denen mit einer Stützplatte (103) gearbeitet wird) am Werkstück seitlich das Master-Bauteil (101) freigelegt, so dass es ggf. leicht von der Stützplatte (103) zu trennen ist (je nach dem gegebenenfalls zu Beginn bei der Fixierung des Master-Bauteils (101) in der Stützplatte (103) verwendeten Klebstoffs kann die Stützplatte (103) kann unter Aufwendung von geringen Zugkräften abgenommen werden oder der Klebstoff wird unter der Einwirkung von Wärme oder Naßchemie gelöst).

Auch ohne eine Ablösung der Stützplatte (103) ist das Master-Bauteil (101) durch seine zumindest teilweise randseitige Freilegung seitlich für die Einwirkung von Nasschemie zugänglich. Vorzugsweise wird die Metallschicht (106) vom Master-Bauteil (101) separiert, indem vorzugsweise das Master-Bauteil (101) nasschemisch (beispielsweise mittels einer KOH-Lösung) aufgelöst wird (siehe Fig. 4i). Auf diese Weise erhält man eine Metallplatte oder Metallfolie (106f) mit komplementären Mikrostrukturen (106b).

Falls vorhanden wird auch die vormals aufgebrachte Haftschicht aus Chrom weggeätzt. Optional wird die Metallfolie (106f) vor Einbau in ein Abformwerkzeug an ihren Rändern noch weiter bearbeitet (siehe Fig. 4j, 4k und 4l), um beispielsweise Grate (106g) zu entfernen, die zuvor durch aufeinanderfolgendes Markieren und Sägen (schematisch dargestellt in Fig. 4f bis 4g) entstanden sind. Dies ist beispielsweise dann zweckdienlich, wenn die Randgeometrie der Metallfolie (106f) oder insbesondere einer entsprechend strukturierten, selbsttragenden Metallplatte von Bedeutung beim Aufbau des späteren Abformwerkzeugs ist (d.h. im Falle einer später lediglich einzuklemmenden Folie ist eine solche Weiterbearbeitung in der Regel nicht nötig). Eine solche Randbearbeitung kann beispielsweise auch dann komplett entfallen, wenn ein leitendes Material (Metall oder hochdotierter Halbleiter) für das Master-Bauteil (101) verwendet wird. In dem Fall kann die galvanisch abgeschiedene Metallschicht (106) für die Ausbildung von geeigneten Rändern bzw. gewünschten Randformen in einem Senkerodier-Prozess über dem leitenden Master-Bauteil (101) an den durch die Kontur (107) vorgegebenen Stellen komplett durchtrennt werden bzw. es kann in das Material des Master-Bauteils (101) hinein erodiert werden.

Damit die komplementären Mikrostrukturen (106b) in dem Fall, wo eine Randbearbeitung benötigt wird, bei dieser Randbearbeitung geschützt sind, werden sie zuvor mit einer Schutzschicht (109) (beispielsweise Photolack) bedeckt (siehe Fig. 4j). Dann werden in gewünschter Weise die Kanten der Metallfolie (106f) bearbeitet, z.B. indem ein nach dem Sägen verbleibender Grat (106g) vorzugsweise durch Abschleifen entfernt wird oder die Kanten insgesamt abgeschliffen werden (Ergebnis siehe Fig. 4k). Nach dieser Bearbeitung wird die Schutzschicht (109) z.B. nasschemisch wieder entfernt (Ergebnis siehe Fig. 4l).

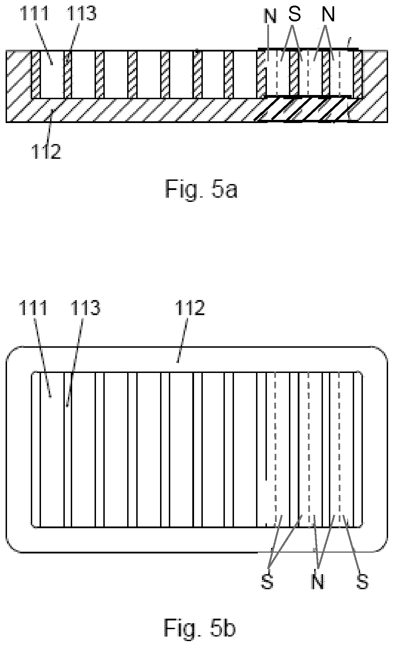

Zur Verwendung der erzeugten Metallplatte oder Metallfolie (106f) in einem Abformwerkzeug (110) wird diese vorzugsweise magnetisch auf einer Halteplatte (112) angebracht. Für die magnetische Anbringung auf der Halteplatte (112) muss die Metallplatte oder Metallfolie (106f) ferromagnetisch sein, was nach der hier bevorzugten galvanischen Abscheidung von Nickel, Nickelcobalt oder Nickeleisen zur Erzeugung der Metallplatte oder Metallfolie (106f) der Fall ist. Bevorzugt ist die Halteplatte (112) selbst magnetisch oder beinhaltet Magnete (111) bzw. stellt eine Aufnahme für Magnete (111) dar. Im Ausführungsbeispiel, so wie es in Fig. 4m und 4n und Fig. 5 gezeigt ist weist die Halteplatte (112) (vorzugsweise aus Metall, beispielsweise aus Stahl oder Messing) mindestens eine Vertiefung für die Aufnahme eines mindestens einen Magneten (111) auf. In einem erfindungsgemäßen Abformwerkzeug wird die Metallplatte oder Metallfolie jedoch stets durch mehrere Magnete gehalten, welche wie weiter unten beschrieben derart angeordnet sind, dass sich jeweils nebeneinander angeordnete Magnete hinsichtlich ihrer Polungsrichtung abwechseln. Bevorzugt sind in der genannten mindestens einen Vertiefung mehrere Magneten (111) mit dazwischen angeordneten Trennelementen wie beispielsweise Trennblechen (113) angeordnet. Alternativ weist die Halteplatte (112) mehrere Vertiefungen für mehrere Magnete (111) auf, die durch schmale Stege getrennt werden. Die Maße der Magnete (111) und Vertiefungen (und falls vorhanden Trennelemente oder Stege) sind derart gewählt bzw. auf einander abgestimmt, dass nach Einsetzen der Magnete (111) in die Vertiefungen die Halteplatte (112) und die Magnete (111) eine ebene Fläche bilden, auf welche die Metallplatte oder Metallfolie (106f) mit ihrer ebenen Oberfläche (106a) plan und vorzugsweise direkt aufgelegt wird.

Auf diese Weise wird die Metallfolie (106f) bzw. die Metallplatte mit den komplementären Mikrostrukturen(106b) über magnetische Kräfte im Abformwerkzeug (110) gehalten (auch beim Entformungsprozess).

Bevorzugt wird ein Spritzgussprozess, besonders bevorzugt ein Kunststoffspritzgussprozess, als Abformprozess bei der Herstellung der mikrofluidischen Bauteile bzw. Düsen verwendet. Bei so einem Spritzgussprozess werden in der Regel hohlförmige Abformwerkzeuge verwendet, die beim Einleiten von verflüssigtem Material bzw. Kunststoff geschlossen sind und nach Erstarren des Materials zur Entnahme des gefertigten Bauteils bzw. des Mikrostrukturen aufweisenden Grundkörpers geöffnet werden. Dieser Art ist auch das Abformwerkzeug (110), das in Fig. 4n schematisch dargestellt ist.

Im Darstellungsbeispiel (siehe Fig. 4n) wird vorzugsweise die Metallfolie (106f) im geschlossenen Abformwerkzeug (110) auch durch einen Formschluss gehalten bzw. dadurch fixiert, dass die Metallfolie (106f) eingeklemmt wird. Dieses Verklemmen der Metallplatte oder der Metallfolie (106f) entsteht bei geschlossenem Abformwerkzeug (110) vorzugsweise randseitig zwischen der Halteplatte (112) und einem weiteren, schalenartigen Werkzeugteil (110a).

Durch die Befestigung der Metallfolie (106f) bzw. der Metallplatte mit den komplementären Mikrostrukturen (106b) nur mittels magnetischer Kräfte und Verklemmung kann sie nach Abnutzung aus dem geöffneten Werkzeug leicht gelöst und ersetzt werden. Im Falle der Verwendung von dickeren (selbsttragenden) strukturierten Metallplatten, deren Herstellung im Vorfeld vergleichsweise lange Galvanik-Prozesszeiten benötigt, können diese auch statt magnetischer Halterung mittels Formschluss an geeigneten Konturen befestigt werden. Diese Variante gehört jedoch nicht zur vorliegenden Erfindung.

Das Werkzeug ist beim Abformprozess hohen Temperaturen und dementsprechend auch starken Temperaturschwankungen ausgesetzt. In diesem Zusammenhang ist die Verwendung von nickelhaltigen Metallfolie (106f) und insbesondere von Metallfolien (106f) aus Nickel-Cobalt aufgrund ihrer Stabilität bei den üblicherweise bei Abformprozessen verwendeten Temperaturen bevorzugt (die Werkzeugtemperaturen liegen bei den hier betrachteteten Kunststoffen typischerweise in Bereichen bis 190°C).

Je nach Schmelztemperatur des beim Spritzguss-Prozess verwendeten Materials bzw. Kunststoff ist das Abformwerkzeug (110) und damit auch die Halterung der Metallplatte bzw. der Metallfolie (106f) hohen Temperaturen ausgesetzt. Das unter Umständen bei Temperaturen von 190 bis 350°C verflüssigte Kunststoffmaterial wird beim Spritzguss-Prozess in das Abformwerkzeug (110) bzw. in die vom Abformwerkzeug (110) gebildete Hohlform eingeleitet, wo es erstarrt. Dieser Prozess kann optional auch variotherm gefahren werden, was eine Steuerung der Werkzeugtemperatur bedingt. Nach der Erstarrung des Kunststoffs im Abformwerkzeug (110) wird dieses bzw. die vom Abformwerkzeug (110) gebildete Hohlform geöffnet und der die Mikrostrukturen aufweisende Grundkörper entnommen. Hierzu müssen sogenannte Entformungskräfte aufgebracht werden, insbesondere bedeutet dies, dass hier ein gewisser Kraftaufwand nötig ist, um den Grundkörper an seiner strukturierten Seite aus den formgebenden Werkzeugstrukturen (also den komplementären Mikrostrukturen (106b) auf der Metallfolie (106f)) zu lösen. Die Halterung der Metallfolie (106f) im Abformwerkzeug (110) muss also derart robust sein, dass sie den auftretenden Entformungskräften widersteht, d.h. Haltkräfte der Magneten (111) müssen also auch größer als die auftretenden Entformungskräfte sein. Damit die Metallfolie (106f) auch beim Entformen der gespritzten Teile aus dem noch heißen Abformwerkzeug (110) in Position gehalten wird, werden vorzugsweise Magnete (111) aus Samarium-Cobalt (SmCo) verwendet, deren magnetische Kräfte auch bei höheren Temperaturen als beispielsweise bei Ferritmagneten oder NdFeB-Magneten vorhanden sind (bei bis ca. 200°C behalten die SmCO-Magnete ihre Magnetkraft).

Erstaunlicherweise hat es sich gezeigt, dass die magnetische Haltekraft, die SmCo-Magnete auf eine ferromagnetische Metallplatte oder eine insbesondere nickelhaltige Metallfolie (106f) ausüben, dadurch verstärkt werden kann, dass mehrere SmCo-Magnete verwendet und dabei so angeordnet werden, dass ihre Polung abwechselnd ausgerichtet ist.

So konnten die magnetischen Haltekräfte, die auf die ferromagnetische Metallplatte oder Metallfolie (106f) wirken, durch den Einsatz mehrere Magnetkörper mit solch einer abwechselnde Anordnung der Pole verstärkt werden. Insbesondere werden hierbei für die Magnete (111) rechteckige, plattenförmige SmCO-Magnete in paralleler Anordnung verwendet, wobei die flächigen Seiten die Pole aufweisen und die Anordnung so alternierend ist, dass jeweils zwei gleiche Polarten zu einander zeigen, wobei sie vorzugsweise durch Abstandshalter voneinander getrennt sind. Eine derartige, bevorzugte Anordnung ist beispielsweise in Fig. 5 zu sehen. Die plattenförmigen Magnete (111) sind auf einer Halteplatte (112) angeordnet. Bevorzugt weist die Halteplatte (112) eine Vertiefung auf, die durch die Magnete (112) und optionale Abstandshalter zwischen den Magneten (112) ausgefüllt ist, wobei die Vertiefung derart bündig mit Magneten (111) und ggf. Abstandshaltern aufgefüllt ist, dass sich eine ebene Oberfläche an der Oberseite (d.h. an der die Vertiefung aufweisenden Seite der Halteplatte (112)) der Halteplatte ergibt. Die Metallplatte mit den komplementären Mikrostrukturen bzw. die Metallfolie (106f) wird vorzugsweise direkt auf diese Oberfläche aufgelegt. Auf diese Weise bilden die sich in der Polungsrichtung abwechselnden Magnete (111), gegebenenfalls zusammen mit den Kanten von optional zwischen den Magneten angeordneten Abstandshaltern, eine Auflagefläche für die Metallplatte oder Metallfolie (106f). Als Abstandshalter werden vorzugsweise Trennblechen (113) verwendet, die optional ferromagnetisch sind. Bevorzugt sind die Trennbleche (113) wie die Magnete (111) rechteckig und plattenförmig, wobei die Breite und Länge der Platten identisch zu denen der Magnete (111) ist. Lediglich die Dicke der Trennbleche (113) unterscheidet sich von der Dicke der plattenförmigen Magnete (111): Vorzugsweise sind die Trennbleche (113) dünner als die Magnete (111) und weisen nur maximal die gleiche Plattendicke auf.

Vorzugsweise sind Halteplatte (112), Magnete (111) und Abstandhalter (wenn vorhanden) miteinander verklebt oder anderweitig untrennbar miteinander verbunden.

Alternativ zum in Fig. 4n angedeuteten Spritzguss-Abformprozess können die Grundkörper (welche die Mikrostrukturen aufweisen und Halbzeuge bei der Fertigung der mikrofluidischen Bauteile darstellen) auch in anderen Abformprozessen hergestellt werden, beispielsweise in einem Heißprägeprozess oder in einem Spritzprägeprozess (wie beispielsweise aus der Herstellung von CDs oder DVDs bekannt; ein mögliche Prozesse ist z.B. in der EP0537953A2 beschrieben), in denen dann vorschlagsgemäß hergestellte komplementäre Mikrostrukturen (106b) verwendet werden. Spritzprägeprozesse werden hier allerdings nur bevorzugt bei der Erzeugung von mikrofluidischen Bauteilen eingesetzt, wenn diese lediglich geringe Strukturtiefen, insbesondere Strukturtiefen kleiner 10µm, vorzugsweise kleiner 5µm aufweisen. Für die Erzeugung von mikrofluidischen Bauteilen mit größeren Strukturtiefen sollte ein anderer Abformprozess (beispielsweise Kunststoff-Spritzguss) gewählt werden. Bei einem Heißprägeprozess wird beispielsweise ein Kunststoff-Rohling in die Abformanlage eingelegt, darin aufgeheizt (allerdings nicht notwendigerweise bis zur Verflüssigung des Kunststoffs) und ein Stempel mit den komplementären Mikrostrukturen (106b) auf den aufgeheizten Rohling gedrückt. Nach Abkühlen des so erzeugten Grundkörpers wird der Stempel zurückgefahren und das Bauteil entnommen.

Bevorzugt weist der in einem Kunststoff-Abformprozess (Spritzgussprozess, Spritzprägeprozess oder Heißprägeprozess) erzeugte Grundkörper mindestens eine vorzugsweise aber mehrere Mikrostrukturengruppen (102) auf, die in Form von Gräben auf einer Grundplatte ausgebildet sind. Bezogen auf das herzustellende mikrofluidische Bauteil bzw. auf die Düse (12) im Anwendungsbeispiel bildet diese Grundplatte oder ein eine Mikrostrukturgruppe (102) umfassender Teil dieser Grundplatte die mikrostrukturierte Platte (12a), die von einem Deckel (12b) bedeckt wird (siehe Fig. 4o). Der Deckel (12b) ist vorzugsweise plattenförmig oder ist zumindest auf der zur Grundplatte bzw. Platte (12a) zugewandten Seite eben. Durch die Deckelung ergeben sich entlang ihrer Länge geschlossene Kanalstrukturen, wobei die Gräben des im Kunststoff-Abformprozess erzeugten Bauteil drei Wände eines rechteckförmigen Kanals bilden und die vierte Wand durch den Deckel (12b) gebildet wird.

Bevorzugt wird die Grundplatte auf ihrer mikrostrukturierten Seite mit einem Deckel (12b) bedeckt, der alle Mikrostrukturgruppen (102) auf der Grundplatte gemeinsam abdeckt, wobei Deckel (12b) und Grundplatte vorzugsweise untrennbar miteinander verbunden werden. Diese Verbindung erfolgt beispielsweise durch Thermokompressionsbonden, Laserbonden, Plasma-aktiviertes Bonden oder besonders bevorzugt Lösungsmittelbonden. Durch diese Verbindungstechniken können Verbindungen zwischen Grundplatte und Deckel erzeugt werden, die einem Flüssigkeits-Innendruck (beispielsweise von bis zu 1000 bar) wie beispielsweise in dem Zerstäuber (1) aus dem Anwendungsbeispiel widerstehen können. Für mikrofluidische Bauteile, in denen die Flüssigkeit unter vergleichsweise geringem Druck geführt wird, ist auch die Verwendung eines Deckels in Form einer auflaminierten, aufgesiegelten oder aufgeklebten Folie möglich.

Bevorzugt weist die Grundplatte zusätzlich zu den Mikrostrukturgruppen (102) auch insbesondere in Randnähe Strukturen auf, die als Markierungen dienen. Dies können beispielsweise Kennzeichnungen der Strukturart und/oder vorzugsweise sogenannte Schneidmarkierungen sein. Bevorzugt enthält die gedeckelte Grundplatte eine Vielzahl von Mikrostrukturgruppen (102), die erst durch ein Abtrennen voneinander vereinzelt werden. Das Abtrennen erfolgt vorzugsweise durch Sägen. Die Schneidmarkierungen ermöglichen das präzise Ansetzen von Sägeschnitten, mit denen die gedeckelte Grundplatte in die einzelnen mikrofluidischen Bauteile (bzw. Düsen) zerlegt wird. Im Falle der Herstellung der Düsen (12) im Anwendungsbeispiel ist diese Präzision beim Sägen wichtig, da erst beim Sägen an den Sägekanten die Düsenöffnungen (12e) und die Einlassöffnungen (12g) freigelegt werden.

Die geschilderten Herstellverfahren sind auf die Herstellung vieler Arten von mikrofluidischen Bauteilen für viele Arten von Geräte übertragbar, in denen Flüssigkeiten gefördert oder transportiert werden. Der vorschlagsgemäße Zerstäuber (1) arbeitet zwar insbesondere rein mechanisch. Der Einsatz solcher mikrofluidischer Bauteile ist jedoch nicht auf die Anwendung in rein mechanischen Geräten zur Ausbringung einer Flüssigkeit begrenzt. Es kann beispielsweise ebenso in Systemen eingesetzt werden, in denen die Ausbringung der Flüssigkeit durch Treibgas oder durch elektrische, hydraulische oder andere Pumpen angetrieben ist. Begriffe wie "Druckerzeuger" sind also allgemein zu verstehen. In diesem Sinne können derartige mikrofluidische Bauteile auch Gebiets-übergreifend verwendet werden; selbst Anwendungen über den medizinischen Bereich hinaus sind möglich.

Der hier dargestellte Zerstäuber dient zwar speziell der Ausgabe einer flüssigen medizinischen Formulierung als inhalierfähiges Aerosol und eignet sich für die Ausbringung sowohl wässriger als auch beispielsweise alkoholischer, insbesondere ethanolischer medizinischer Formulierungen.

Bevorzugte Inhaltsstoffe der vorzugsweise flüssigen medizinischen Formulierung sind insbesondere in den Schriften WO09/047173A2 und WO09/115200A1 aufgeführt, die hiermit diesbezüglich vollumfänglich als Referenz eingeführt werden. Insbesondere kann es sich bei dem in diesen Schriften beschriebenen Fluid um wässrige oder nicht wässrige Lösungen, Mischungen, Formulierungen mit und ohne Lösungsmittel, wie Ethanol oder dergleichen, handeln.

**Bezugszeichenliste**

| | | | |
|---|---|---|---|
| 1 | Zerstäuber | 40 | Taste |
| 2 | Flüssigkeit | | |
| 3 | Behälter | 101 | Master-Bauteil |
| 4 | Beutel | 101a | Dicke (des Master-Bauteils) |
| 5 | Druckerzeuger | 102 | Mikrostrukturgruppe |
| 6 | Halterung (für Behälter) | 103 | Stützplatte |
| 7 | Antriebsfeder | 103a | Vertiefung (in Stützplatte) |
| 8 | Sperring | 103b | Rand (der Vertiefung in Stützplatte) |
| 9 | Hohlkolben | | |
| 10 | Rückschlagventil | 104 | Füllmasse |
| 11 | Druckkammer | 105 | Metallisierungsschicht |
| 12 | Düse | 106 | Metallschicht |
| 12a | Platte | 106a | ebene Oberfläche (der Metallschicht) |
| 12b | Deckel | | |
| 12c | Einströmbereich | 106b | komplementäre Mikrostruktur |
| 12d | Düsenkanäle | 106c | Spalt (in Metallschicht) |
| 12e | Düsenöffnungen | 106g | Grat (an Metallfolie) |
| 12f | Feinstfilter | 106f | Metallfolie |
| 12g | Einlassöffnungen | 107 | Elektrode |
| 13 | Mundstück | 107a | vorstehende Kontur (an Elektrode) |
| 14 | Aerosol | | |
| 15 | Zuluftöffnung | 108 | Sägelinie |
| 16 | Gehäuseoberteil | 109 | Schutzschicht |
| 17 | Gehäuseinnenteil | 110 | Abformwerkzeug |
| 18 | Gehäuseunterteil | 110a | schalenartiges Werkzeugteil |
| 19 | Sicherheitsverschluss | 111 | Magnet |
| 20 | Feder | 112 | Halteplatte |
| 21 | Behälterboden | 113 | Trennblech |
| 22 | Anstechelement | | |

## Patentansprüche

1. Abformwerkzeug (110), das als Teil des Abformwerkzeugs (110) eine Metallplatte mit komplementären Mikrostrukturen (106b) oder eine Metallfolie (106f) mit komplementären Mikrostrukturen (106b) beinhaltet,
wobei die Metallplatte oder Metallfolie (106f) durch eine Metallschicht (106) gebildet wird, die über einem Master-Bauteil (101) in einem galvanischen Abscheidungsprozess erzeugt und vom Master-Bauteil (101) getrennt wird, und wobei die komplementären Mikrostrukturen (106b) komplementär zu Mikrostrukturen auf dem Master-Bauteil (101) sind,
**dadurch gekennzeichnet,**
**dass** die Metallplatte oder die Metallfolie (106f) aus einem ferromagnetischen Material besteht und zumindest teilweise magnetisch im Werkzeug gehalten wird, wobei die Metallplatte oder Metallfolie (106f) durch mehrere Magnete (111) gehalten wird, welche derart angeordnet sind, dass sich jeweils nebeneinander angeordneten Magneten (111) hinsichtlich ihrer Polungsrichtung abwechseln.

2. Abformwerkzeug (110) nach Anspruch 1 **dadurch gekennzeichnet, dass** die Metallplatte oder die Metallfolie (106f) aus Nickel oder Nickeleisen oder Nickel-Cobalt besteht oder Nickel enthält.

3. Abformwerkzeug (110) nach Anspruch 1 oder 2 dadurch charakterisiert, dass die Magnete (111) mehrere vorzugsweise gleichförmige Magnete (111) aus Samariumcobalt sind.

4. Abformwerkzeug (110) nach einem der vorherigen Ansprüche **dadurch gekennzeichnet, dass** die Magnete (111) parallel nebeneinander angeordnet sind.

5. Abformwerkzeug (110) nach Anspruch 4 **dadurch gekennzeichnet, dass**
die Magnete (111) rechteckig und plattenförmig sind,
die flächigen Seiten der Magnete (111) die Pole aufweisen,
die Anordnung der Magnete (111) derart alternierend ist, dass jeweils zwei gleiche Polarten zueinander zeigen und
die Magnete (111) vorzugsweise durch Abstandshalter voneinander getrennt sind.

6. Abformwerkzeug (110) nach einem der vorangegangenen Ansprüche, wobei die Metallplatte oder die Metallfolie (106f) in einem galvanischen Abscheidungsprozess über einem mikrostrukturiertes Master-Bauteil (101) aus einem Halbleitermaterial, vorzugsweise aus einkristallinem Silizium, hergestellt ist.

7. Abformwerkzeug (110) nach Anspruch 6 **dadurch gekennzeichnet, dass** die Mikrostrukturen auf dem Master-Bauteil (101) in einem photolithographischen Verfahren insbesondere in Verbindung mit einem ionenunterstützten reaktiven Trockenätzverfahren hergestellt sind.

8. Abformwerkzeug (110) nach einem der vorangegangenen Ansprüche **dadurch gekennzeichnet, dass** das Abformwerkzeug (110) eine Vielzahl von komplementären Mikrostrukturgruppen aufweist.

9. Abformwerkzeug (110) nach Anspruch 8 **dadurch gekennzeichnet, dass** die komplementären Mikrostrukturgruppen in parallelen Reihen und Spalten angeordnet sind.

10. Abformwerkzeug (110) nach einem der vorangegangenen Ansprüche **dadurch gekennzeichnet, dass** das Abformwerkzeug (110) ein Werkzeug für einen Heißprägeprozess oder einen Spritzprägeprozess oder einen Spritzgussprozess ist.

11. Abformwerkzeug (110) nach einem der vorangegangenen Ansprüche, **dadurch gekennzeichnet, dass** das Abformwerkzeug (110) ein Werkzeug zur Abformung von Kunststoff ist, wobei der Kunststoff ein COC, COP, Polystyrol, Polypropylen, PC, PEEK, PMP oder PMMA ist.

12. Verwendung des Abformwerkzeugs nach Anspruch 1 zur Herstellung eines mikrofluidischen Bauteils, wobei das mikrofluidische Bauteil eine Platte (12a) mit grabenartigen Mikrostrukturen aufweist, die längsseitig von einem Deckel (12b) bedeckt sind, und wobei die Platte (12a) und der Deckel (12b) aus Kunststoff bestehen und fest miteinander verbunden sind, wobei die Platte (12a) mit den Mikrostrukturen in einem Abformprozess mit dem Abformwerkzeug erzeugt wird.

## Claims

1. Moulding tool (110), which as part of the moulding tool (110) includes a metal plate with complementary microstructures (106b) or a metal foil (106f) with complementary microstructures (106b),
the metal plate or metal foil (106f) being formed by a metal layer (106) which is produced by means of a master component (101) in an electrodeposition process and is separated from the master component (101), and the complementary microstructures (106b) being complementary to microstructures on the master component (101),
**characterised in that** the metal plate or the metal foil (106f) is made of a ferromagnetic material and is retained at least partially magnetically in the tool, the metal plate or metal foil (106f) being retained by a plurality of magnets (111) which are arranged in such a way that in each case the magnets (111) arranged adjacent to one another alternate with regard to their polarisation direction.

2. Moulding tool (110) according to claim 1, **characterised in that** the metal plate or the metal foil (106f) is made of nickel or nickel iron or nickel cobalt, or contains nickel.

3. Moulding tool (110) according to either claim 1 or claim 2, **characterised in that** the magnets (111) are a plurality of preferably uniform magnets (111) made of samarium cobalt.

4. Moulding tool (110) according to any one of the preceding claims, **characterised in that** the magnets (111) are arranged in parallel adjacent to one another.

5. Moulding tool (110) according to claim 4, **characterised in that**
the magnets (111) are rectangular and plate-shaped,
the flat sides of the magnets (111) have the poles,
the arrangement of the magnets (111) is alternating so that in each case two of the same pole types are directed towards one another, and
the magnets (111) are preferably separated from one another by spacers.

6. Moulding tool (110) according to any one of the preceding claims, wherein the metal plate or the metal foil (106f) is produced in an electrodeposition process by means of a microstructured master component (101) made of a semiconductor material, preferably of monocrystalline silicon.

7. Moulding tool (110) according to claim 6, **characterised in that** the microstructures on the master component (101) are produced in a photolithographic process, preferably in conjunction with an ion-assisted reactive dry etching method.

8. Moulding tool (110) according to any one of the preceding claims, **characterised in that** the moulding tool (110) has a multiplicity of complementary microstructure groups.

9. Moulding tool (110) according to claim 8, **characterised in that** the complementary microstructure groups are arranged in parallel rows and columns.

10. Moulding tool (110) according to any one of the preceding claims, **characterised in that** the moulding tool (110) is a tool for a hot stamping process or a compression injection moulding process or an injection moulding process.

11. Moulding tool (110) according to any one of the preceding claims, **characterised in that** the moulding tool (110) is a tool for moulding plastics material, the plastics material being a COC, COP, polystyrene, polypropylene, PC, PEEK, PMP or PMMA.

12. Use of the moulding tool according to claim 1 for producing a microfluidic component, wherein the microfluidic component has a plate (12a) with groove-like microstructures which are covered on the long side by a cover (12b), and wherein the plate (12a) and the cover (12b) are made of plastics material and are firmly connected to one another, wherein the plate (12a) with the microstructures is produced by means of the moulding tool in a moulding process.

## Revendications

1. Outil de moulage (110), qui contient en tant que partie de l'outil de moulage (110) une plaque métallique avec des microstructures complémentaires (106b) ou une feuille métallique (106f) avec des microstructures complémentaires (106b),
dans lequel la plaque métallique ou feuille métallique (106f) est formée par une couche métallique (106), qui est produite par le biais d'un composant maître (101) dans un procédé de séparation galvanique et séparée du composant maître (101), et dans lequel les microstructures complémentaires (106b) sont complémentaires à des microstructures sur le composant maître (101),
**caractérisé en ce**
**que** la plaque métallique ou la feuille métallique (106f) se compose d'un matériau ferromagnétique et est retenue au moins en partie magnétiquement dans l'outil,
dans lequel la plaque métallique ou feuille métallique (106f) est retenue par plusieurs aimants (111), lesquels sont agencés de sorte que des aimants (111) agencés respectivement l'un à côté de l'autre s'alternent en ce qui concerne leur polarité.

2. Outil de moulage (110) selon la revendication 1 **caractérisé en ce que** la plaque métallique ou la feuille métallique (106f) est constituée de nickel ou de fer nickelé ou de nickel-cobalt ou contient du nickel.

3. Outil de moulage (110) selon la revendication 1 ou 2 **caractérisé en ce que** les aimants (111) sont plusieurs aimants (111) de préférence uniformes, en samarium-cobalt.

4. Outil de moulage (110) selon l'une quelconque des revendications précédentes **caractérisé en ce que** les aimants (111) sont agencés parallèlement l'un à côté de l'autre.

5. Outil de moulage (110) selon la revendication 4 **caractérisé en ce que**
les aimants (111) sont rectangulaires et en forme de plaque,
les côtés plans des aimants (111) présentent les pôles,
l'agencement des aimants (111) est alterné de sorte que respectivement deux polarités identiques sont tournées l'une vers l'autre et
les aimants (111) sont séparés l'un de l'autre de préférence par des écarteurs.

6. Outil de moulage (110) selon l'une quelconque des revendications précédentes, dans lequel la plaque métallique ou la feuille métallique (106f) est fabriquée dans un procédé de séparation galvanique par le biais d'un composant maître (101) microstructuré en un matériau semi-conducteur, de préférence en silicium monocristallin.

7. Outil de moulage (110) selon la revendication 6 **caractérisé en ce que** les microstructures sur le composant maître (101) sont fabriquées dans un procédé photolithographique en particulier en relation avec un procédé de gravure sèche ionique réactive.

8. Outil de moulage (110) selon l'une quelconque des revendications précédentes **caractérisé en ce que** l'outil de moulage (110) présente une pluralité de groupes de microstructures complémentaires.

9. Outil de moulage (110) selon la revendication 8, **caractérisé en ce que** les groupes de microstructures complémentaires sont agencés en rangées et colonnes parallèles.

10. Outil de moulage (110) selon l'une quelconque des revendications précédentes **caractérisé en ce que** l'outil de moulage (110) est un outil pour un procédé d'estampage à chaud ou un procédé d'estampage par injection ou un procédé de moulage par injection.

11. Outil de moulage (110) selon l'une quelconque des revendications précédentes **caractérisé en ce que** l'outil de moulage (110) est un outil pour le moulage de plastique, dans lequel le plastique est un COC, COP, polystyrène, polypropylène, PC, PEEK, PMP ou PMMA.

12. Utilisation de l'outil de moulage selon la revendication 1 pour la fabrication d'un composant microfluidique, dans laquelle le composant microfluidique présente une plaque (12a) avec des microstructures en forme de fossés, qui sont recouvertes côté longitudinal par un couvercle (12b), et dans laquelle la plaque (12a) et le couvercle (12b) sont constitués de plastique et reliés fixement l'un à l'autre, dans laquelle la plaque (12a) avec les microstructures est produite dans un procédé de moulage avec l'outil de moulage.
